# EUROPEAN PATENT SPECIFICATION

(11) **EP 2 556 054 B1**
(45) Date of publication and mention of the grant of the patent: **27.09.2017**
(21) Application number: 11715101.9
(22) Date of filing: 22.03.2011
(51) Int. Cl.: C07C 401/00, C07F 7/18, A61K 31/59, A61P 19/08, A61P 17/06, A61P 37/00

(54) **DIASTEREOMERS OF 2-METHYLENE-19-NOR-22-METHYL-1ALPHA,25-DIHYDROXYVITAMIN D3**
DIASTEREOMERE VON 2-METHYLEN-19-NOR-22-METHYL-1ALPHA,25-DIHYDROXYVITAMIN D3
DIASTÉRÉOISOMÈRES DE 2-MÉTHYLÈNE-19-NOR-22-MÉTHYL-1ALPHA,25- DIHYDROXYVITAMINE D3

(30) Priority: 23.03.2010 US 316653 P
(43) Date of publication of application: 13.02.2013
(73) Proprietor: Wisconsin Alumni Research Foundation, Madison, WI 53707-7365 (US)
(72) Inventor: DELUCA, Hector, F., Deerfield, WI 53531 (US); FLORES, Agnieszka, Madison, WI 53711 (US); GRZYWACZ, Pawel, Madison, WI 53716 (US); PLUM, Lori, A., Arena, WI 53503 (US); CLAGETT-DAME, Margaret, Deerfield, WI 53531 (US); THODEN, James, B., Madison, WI 53711 (US); HOLDEN, Hazel, M., Fitchburg, WI 53711 (US)
(74) Representative: Harrison IP Limited
(86) International application number: PCT/US2011/029452
(87) International publication number: WO 2011/119622

(56) References cited:
- US-A1- 2005 080 059
- Y. SAKAMAKI ET AL.: "Potent antagonist for the vitamin D receptor: vitamin D analogues side chain structure", J. MED. CHEM., vol. 53, 7 July 2010 (2010-07-07), pages 5813-5826, XP002646449,

## Description

### FIELD

This present technology relates to vitamin D compounds, and more particularly to diastereomers of 2-methylene-19-nor-22-methyl-1α,25-dihydroxyvitamin D₃ and derivatives thereof, and to pharmaceutical formulations that include this compound. The present technology also relates to these compounds for use in the treatment of various diseases and in the preparation of medicaments for use in treating various diseases.

### BACKGROUND

The natural hormone, 1α,25-dihydroxyvitamin D₃ (also referred to as 1α,25-dihydroxycholecalciferol and calcitriol) and its analog in the ergosterol series, *i.e.,* 1α,25-dihydroxyvitamin D₂, are known to be highly potent regulators of calcium homeostasis in animals and humans, and their activity in cellular differentiation has also been established, Ostrem et al., Proc. Natl. Acad. Sci. USA, 84, 2610 (1987). Many structural analogs of these metabolites have been prepared and tested, including 1α-hydroxyvitamin D₃, 1α-hydroxyvitamin D₂, various side chain homologated vitamins, and fluorinated analogs. Some of these compounds exhibit an interesting separation of activities in cell differentiation and calcium regulation. This difference in activity may be useful in the treatment of a variety of diseases as renal osteodystrophy, vitamin D-resistant rickets, osteoporosis, psoriasis, and certain malignancies. The structure of 1α,25-dihydroxyvitamin D₃ and the numbering system used to denote the carbon atoms in this compound are shown below. 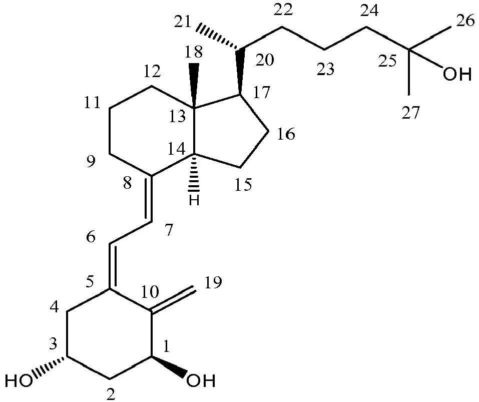 1α,25-Dihydroxyvitamin D₃ = 1α,25-Dihydroxycholecalciferol = Calcitriol

US2005/0080059 discloses a method of preventing and/or treating inflammatory bowel disease, particularly ulcerative colitis and Crohn's disease. The method involves administering a 2-methylene-19-nor-vitamin D compound in an amount effective to treat the disease. This document also teaches that the administration of a 2-methylene-19-nor-vitamin D compound prevents the development of or delays the onset of inflammatory bowel disease in susceptible individuals. In particular, compounds 1alpha-hydroxy-2-methylene-19-nor-homopregnacalciferol and 2-methylene-19-nor-20(S)-1alpha,25-dihydroxyvitamin D3 are disclosed for use in the method referred to in this document.

### SUMMARY

The present technology provides compounds having the structure of formula IA or formula IB as provided herein, pharmaceutical formulations that include these compounds, these compounds for use in treating various disease states using these compounds, and the use of these compounds in the preparation of medicaments for treating various disease states.

Therefore, in one aspect, the present technology provides a compound having the structure of formula IA or of formula IB: 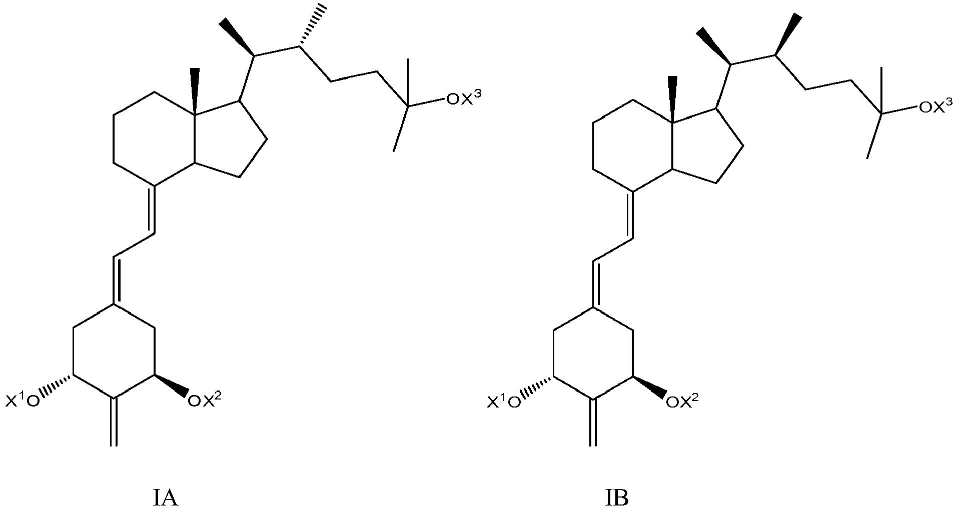 wherein
X¹ and X² are independently selected from H and hydroxyl protecting groups; and
X³ is H.

In some embodiments, X¹ and X² are hydroxy protecting groups such as silyl groups. In some such embodiments, X¹ and X² are both t-butyldimethylsilyl groups. In other embodiments, X¹, X², and X³ are H such that the compound has the formula IIA or IIB as provided herein.

In some embodiments, the compound is (20S, 22R)-2-methylene-19-nor-22-methyl-1α,25-dihydroxyvitamin D₃ and has the formula IIA as shown below, or (20S, 22S)-2-methylene-19-nor-22-methyl-1α,25-dihydroxyvitamin D₃ and has the formula IIB as shown below:

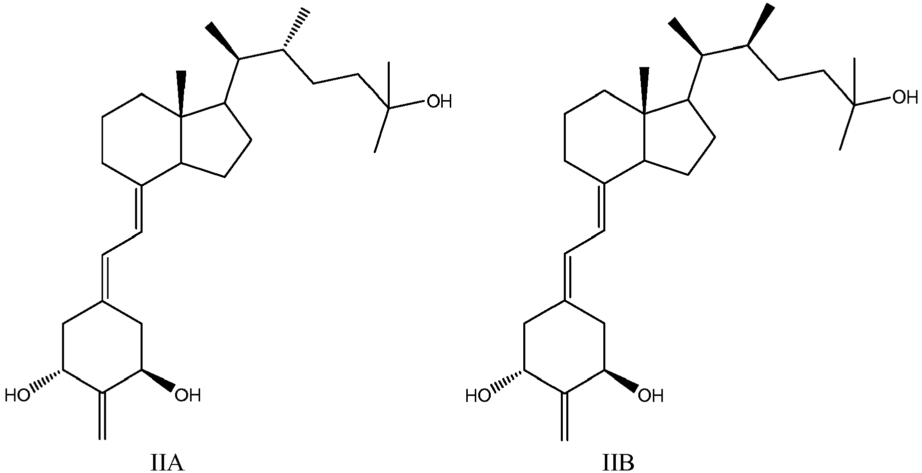

In some such embodiments, the compound of formula IIA is a compound of formula IIE (also known as AGS-1). In other embodiments, the compound of formula IIB is a compound of formula IIF (also known as AGS-2). The structures of formula IIE, IIF are shown below:

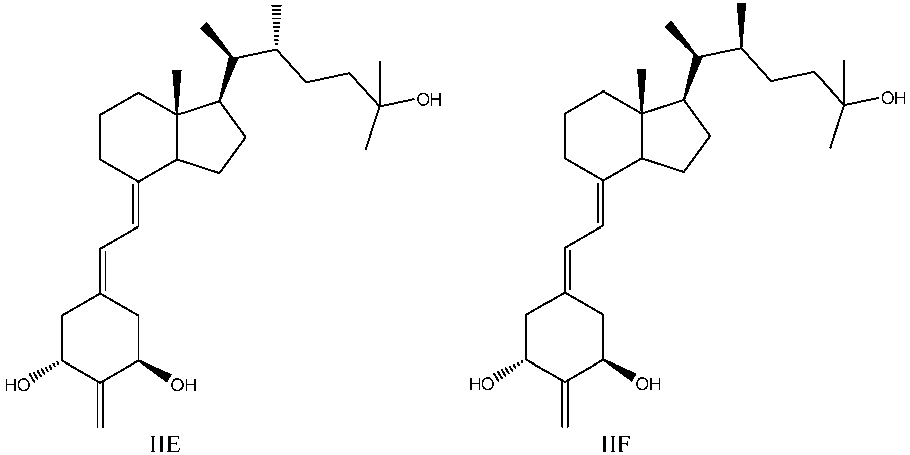

Compounds of the present technology show a highly advantageous pattern of biological activity, including strong binding to the vitamin D receptor and induction of 24-hydroxylase activity. Thus there is provided the present compounds for use in treating a subject suffering from certain biological conditions. The compounds for use may include administering an effective amount of a compound of the present technology to the subject, wherein the biological condition is selected from psoriasis; leukemia; colon cancer; breast cancer; prostate cancer; multiple sclerosis; lupus; diabetes mellitus; host versus graft reaction; rejection of organ transplants; an inflammatory disease selected from rheumatoid arthritis, asthma, or inflammatory bowel diseases; a skin condition selected from lack of adequate dermal hydration, or insufficient sebum secretion; renal osteodystrophy; or osteoporosis.
There is also provided a method comprising administering an effective amount of the compounds of the present technology to a subject suffering from wrinkles or lack of adequate skin firmness.

A compound of the present technology may be present in a composition for use in treating the above-noted diseases and disorders in an effective amount and optionally including a pharmaceutically acceptable carrier. In some embodiments, the amount of compound includes from about 0.01 µg per gram of composition to about 1 mg per gram of the composition, preferably from about 0.1 µg per gram to about 500 µg per gram of the composition, and may be administered topically, transdermally, orally, or parenterally in dosages of from about 0.01 µg per day to about 1 mg per day, preferably from about 0.1 µg per day to about 500 µg per day.

Further features and advantages of the present technology will be apparent from the following detailed description and drawings.

### BRIEF DESCRIPTION OF THE DRAWINGS

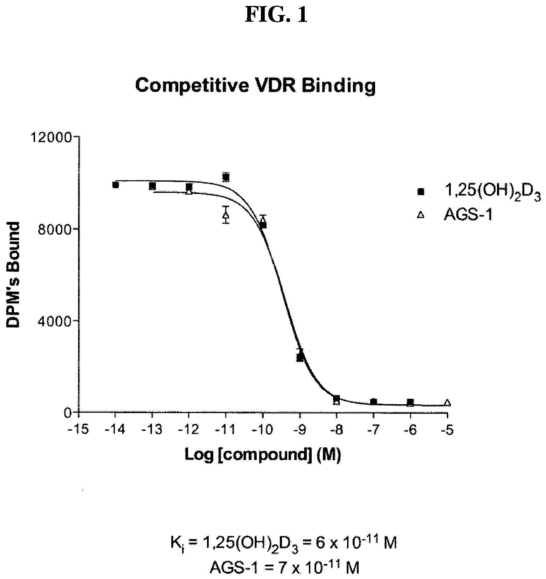

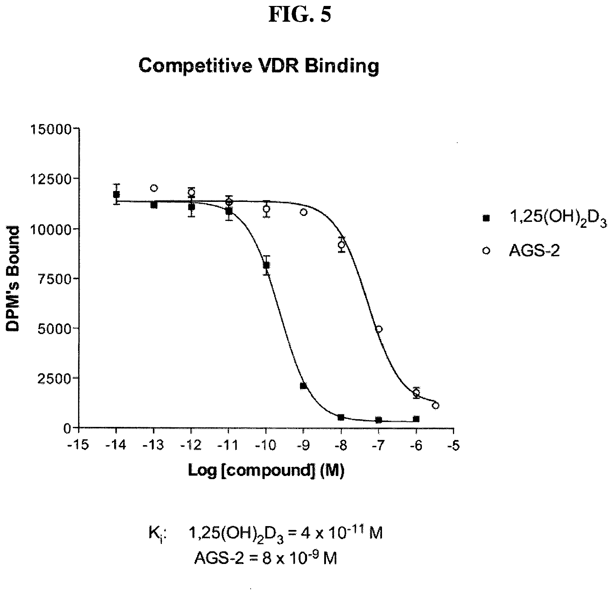

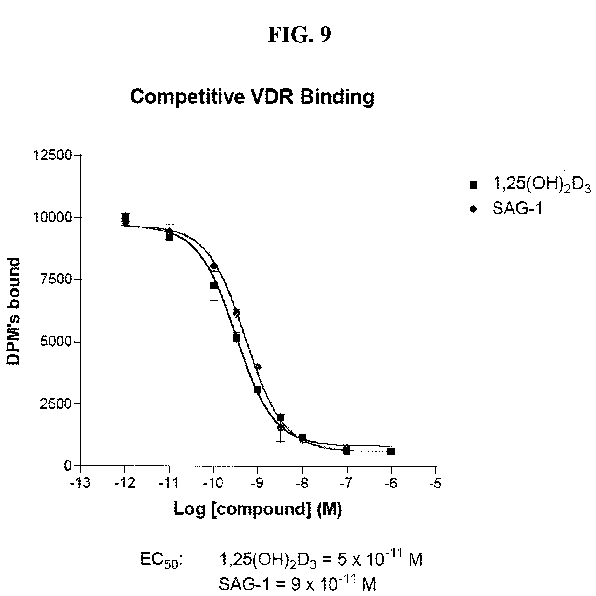

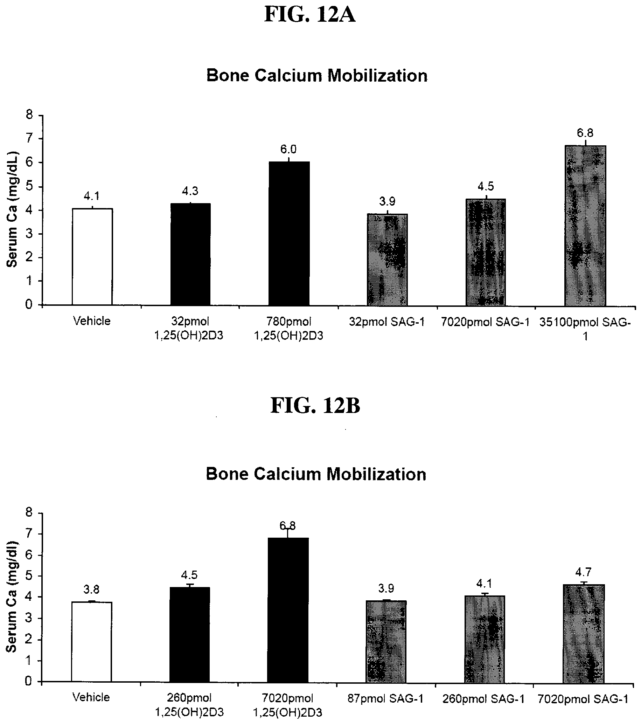

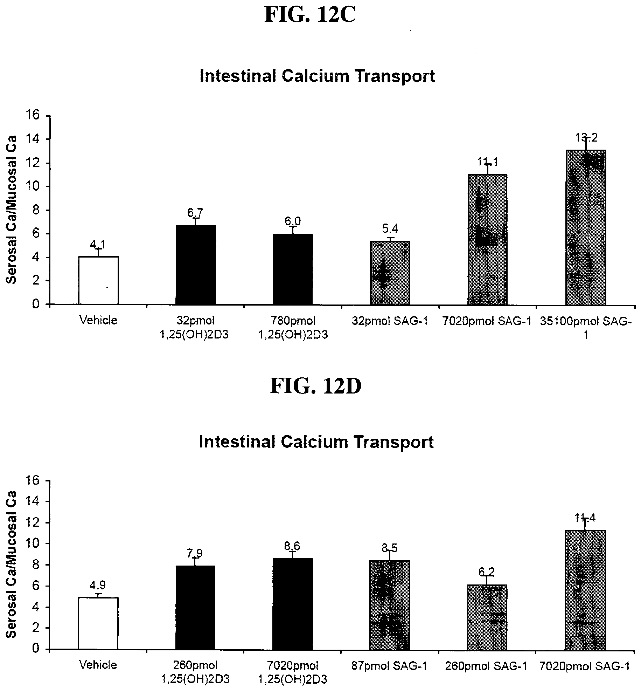

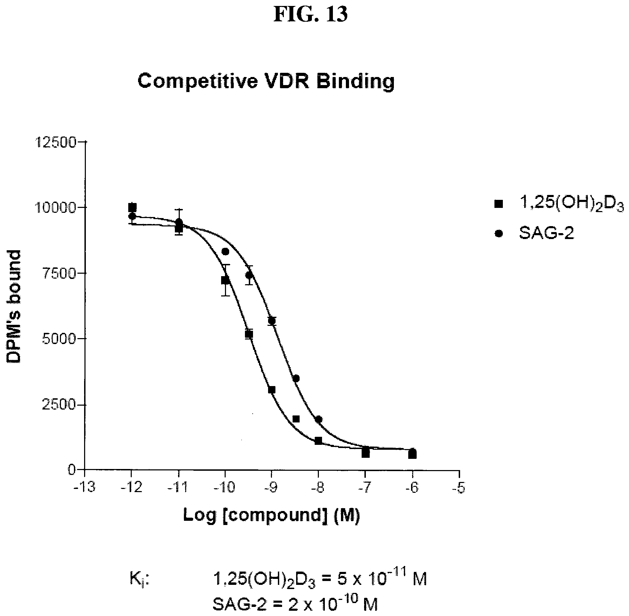

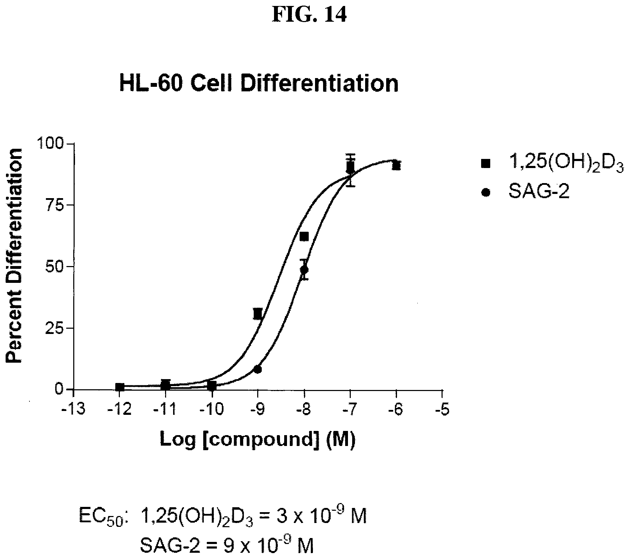

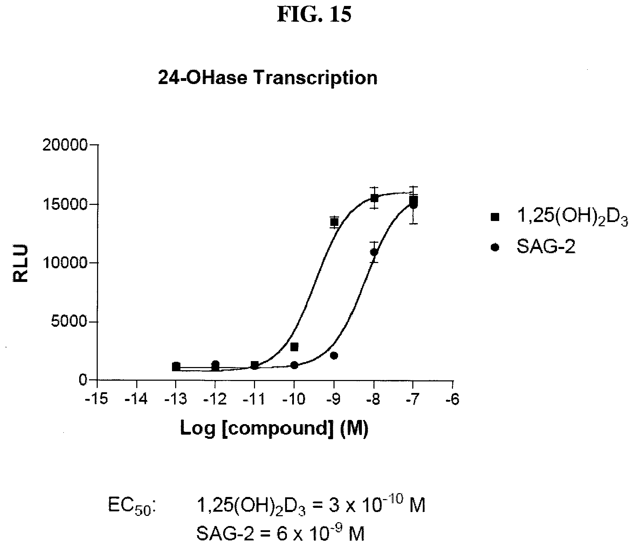

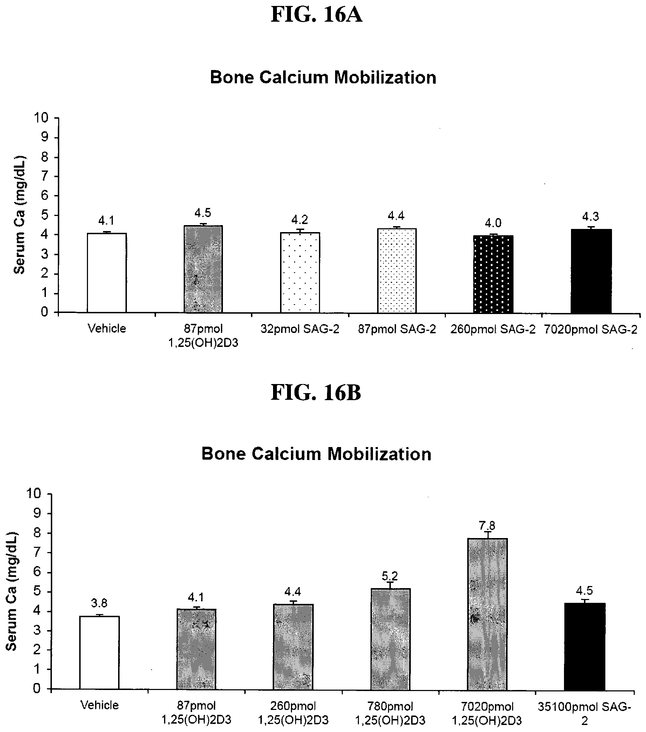

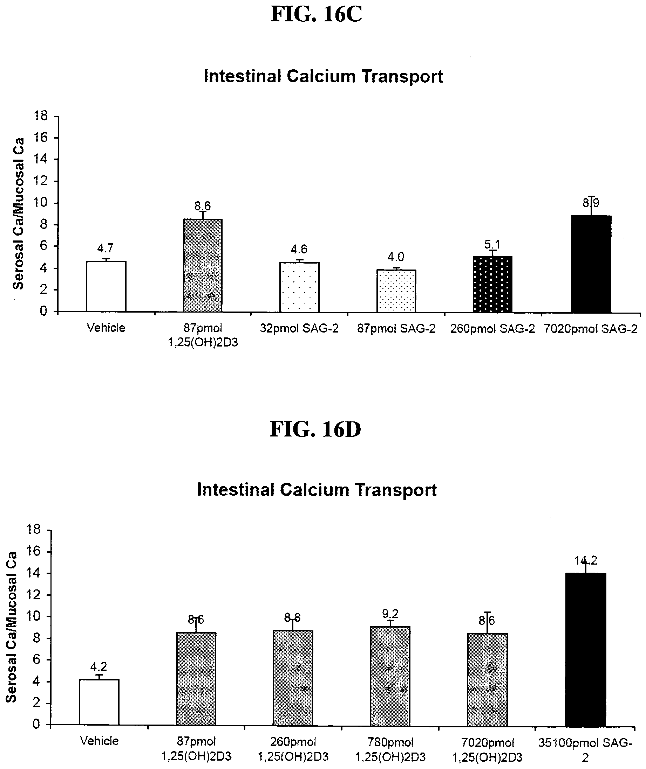

Figures 1-4 illustrate various biological activities of (20S,22R)-2-methylene-19-nor-22-methyl-1α,25-dihydroxyvitamin D₃ (referred to as "AGS-1" in the Figures), compared with those of the native hormone, 1α,25-dihydroxyvitamin D₃ (referred to as "1,25(OH)₂D₃" in the Figures). Figures 5-8 illustrate various biological activities of (20S,22S)-2-methylene-19-nor-22-methyl-1α,25-dihydroxyvitamin D₃ (referred to as "AGS-2" in the Figures) compared with those of the native hormone. Comparative Figures 9-12 illustrate various biological activities of (20R,22S)-2-methylene-19-nor-22-methyl-1α,25-dihydroxyvitamin D₃ (referred to as "SAG-1" in the Figures), compared with those of the native hormone. Comparative Figures 13-16 illustrate various biological activities of (20R,22R)-2-methylene-19-nor-22-methyl-1α,25-dihydroxyvitamin D₃ (referred to as "SAG-2" in the Figures), compared with those of the native hormone.
Fig. 1 shows a graph of competitive binding to the nuclear vitamin D hormone receptor between AGS-1 and the native hormone, 1,25(OH)₂D₃. AGS-1 binds to the nuclear vitamin D receptor with the same affinity as 1,25(OH)₂D₃.
Fig. 2 is a graph comparing the percent HL-60 cell differentiation as a function of the concentration of AGS-1 with that of 1,25(OH)₂D₃. AGS-1 is 300 times more potent as the native hormone in causing the differentiation of HL-60 cells into monocytes.
Fig. 3 is a graph comparing the *in vitro* transcription activity of AGS-1 with that of 1,25(OH)₂D₃. In bone cells, AGS-1 is nearly 40 times more potent than 1,25(OH)₂D₃ in increasing transcription of the 24-hydroxylase gene.
Fig. 4A and Fig. 4B are bar graphs comparing the bone calcium mobilization activity of AGS-1 with that of 1,25(OH)₂D₃ in rat. AGS-1 is both more efficacious and about 10 to 50 times more potent than the native hormone in releasing bone calcium stores. Fig. 4C is a bar graph comparing the intestinal calcium transport activity of AGS-1 with that of 1,25(OH)₂D₃. AGS-1 exhibits higher potency in promoting intestinal calcium transport than the native hormone.
Fig. 5 shows a graph of competitive binding to the nuclear vitamin D hormone receptor between AGS-2 and the native hormone, 1,25(OH)₂D₃. AGS-2 binds to the nuclear vitamin D receptor with lower affinity than 1,25(OH)₂D₃.
Fig. 6 is a graph comparing the percent HL-60 cell differentiation as a function of the concentration of AGS-2 with that of 1,25(OH)₂D₃. AGS-2 is approximately 10 times less potent than the native hormone in causing the differentiation of HL-60 cells into monocytes.
Fig. 7 is a graph comparing the *in vitro* transcription activity of AGS-2 with that of 1,25(OH)₂D₃, in rat osteosarcoma cells. AGS-2 is about 10 times less potent than 1,25(OH)₂D₃ in increasing transcription of the 24-hydroxylase gene.
Fig. 8A is a bar graph comparing the bone calcium mobilization activity of AGS-2 with that of 1,25(OH)₂D₃ in rat. AGS-2 is approximately 50 times less potent than the native hormone in releasing bone calcium stores. Fig. 8B is a bar graph comparing the intestinal calcium transport activity of AGS-1 with that of 1,25(OH)₂D₃. The calcemic activity of AGS-2 in the intestine is similar or greater than the native hormone.
Comparative Fig. 9 shows a graph of competitive binding to the nuclear vitamin D hormone receptor between SAG-1 and the native hormone, 1,25(OH)₂D₃. SAG-1 binds to the nuclear vitamin D receptor with similar or slightly less affinity than 1,25(OH)₂D₃.
Comparative Fig. 10 is a graph comparing the percent HL-60 cell differentiation as a function of the concentration of SAG-1 with that of 1,25(OH)₂D₃. SAG-1 is more than 3 times more potent than the native hormone in causing the differentiation of HL-60 cells into monocytes.
Comparative Fig. 11 is a graph comparing the *in vitro* transcription activity of SAG-1 with that of 1,25(OH)₂D₃. In bone cells, SAG-1 is approximately equal in potency to 1,25(OH)₂D₃ in increasing transcription of the 24-hydroxylase gene.
Comparative Fig. 12A and comparative Fig. 12B are bar graphs comparing the bone calcium mobilization activity of SAG-1 with that of 1,25(OH)₂D₃ in rat. SAG-1 is less potent than the native hormone in releasing bone calcium stores. Comparative Fig. 12C and comparative Fig. 12D are bar graphs comparing the intestinal calcium transport activity of SAG-1 with that of 1,25(OH)₂D₃. SAG-1 exhibits similar potency to the native hormone in transporting calcium across the intestinal epithelium.
Comparative Fig. 13 shows a graph of competitive binding to the nuclear vitamin D hormone receptor between SAG-2 and the native hormone, 1,25(OH)₂D₃. SAG-2 binds to the nuclear vitamin D receptor with approximately 4 times less affinity than 1,25(OH)₂D₃.
Comparative Fig. 14 is a graph comparing the percent HL-60 cell differentiation as a function of the concentration of SAG-2 with that of 1,25(OH)₂D₃. SAG-2 is approximately 3 times less potent than the native hormone in causing the differentiation of HL-60 cells into monocytes.
Comparative Fig. 15 is a graph comparing the *in vitro* transcription activity of SAG-2 with that of 1,25(OH)₂D₃, in rat osteosarcoma cells. SAG-2 is about 20 times less potent than 1,25(OH)₂D₃ in increasing transcription of the 24-hydroxylase gene.
Comparative Fig. 16A and comparative Fig. 16B are bar graphs comparing the bone calcium mobilization activity of SAG-2 with that of 1,25(OH)₂D₃ in rat. SAG-2 has very little to no activity in mobilizing calcium from bone stores. Comparative Fig. 16C and comparative Fig. 16D are bar graphs comparing the intestinal calcium transport activity of SAG-2 with that of 1,25(OH)₂D₃. SAG-2 exhibits less potency compared to the native hormone in transporting calcium across the intestinal epithelium.

### DETAILED DESCRIPTION

(20S,22R)-2-methylene-19-nor-22-methyl-1α,25-dihydroxyvitamin D₃, and (20S,22S)-2-methylene-19-nor-22-methyl-1α,25-dihydroxyvitamin D₃, were synthesized, and tested, and found to be useful in treating a variety of biological conditions as described herein. Structurally, these compounds have the formulas IIA and IIB as shown below. Comparative compounds (20R,22S)-2-methylene-19-nor-22-methyl-1α,25-dihydroxyvitamin D₃, and (20R, 22R)-2-methylene-19-nor-22-methyl-1α,25-dihydroxyvitamin D₃ were also synthesized and tested and these compounds have the formulas IIC and IID as shown below:

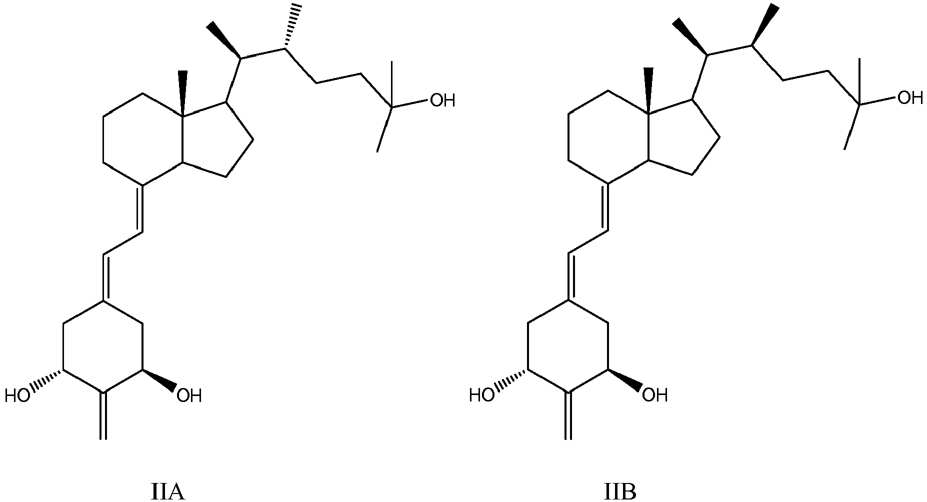

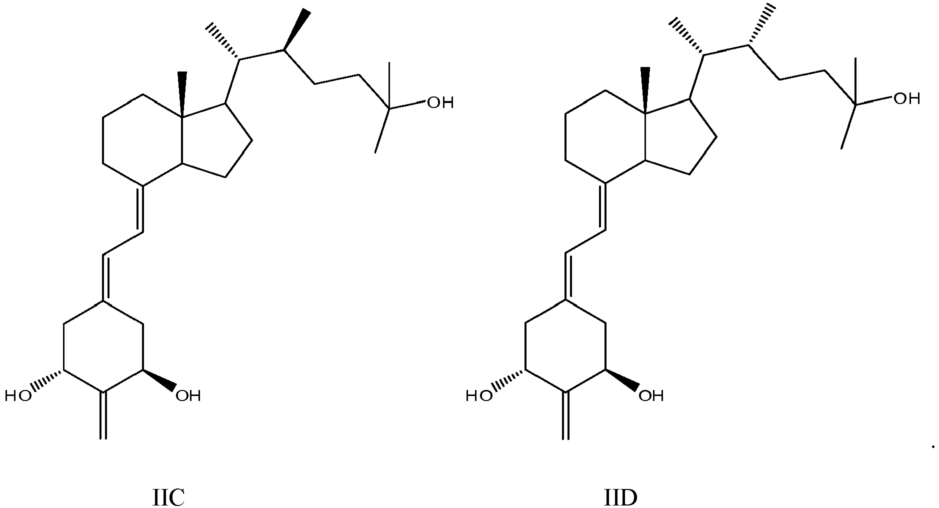

In some such embodiments, the compound of formula IIA is a compound of formula IIE, in other embodiments, the compound of formula IIB is a compound of formula IIF and have the structures shown below:

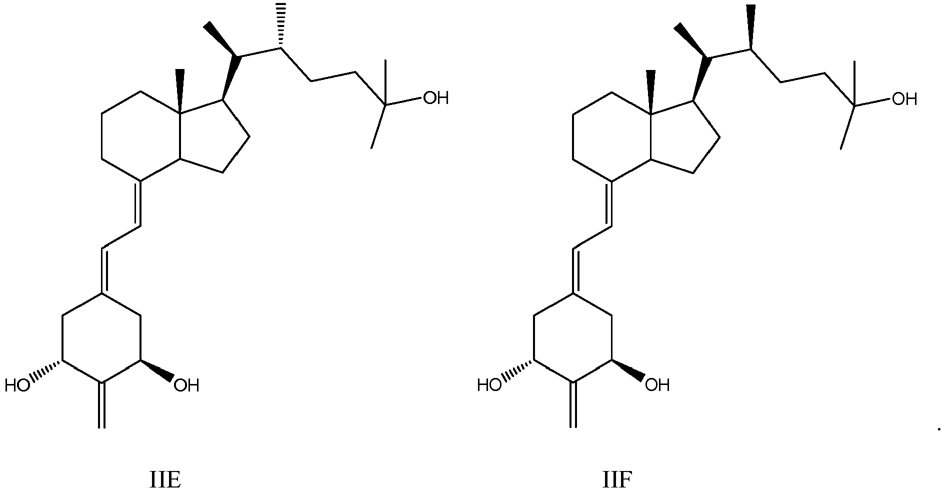

A comparative compound of formula IIG, and a comparative compound of formula IIH having the structures shown below were also synthesised:

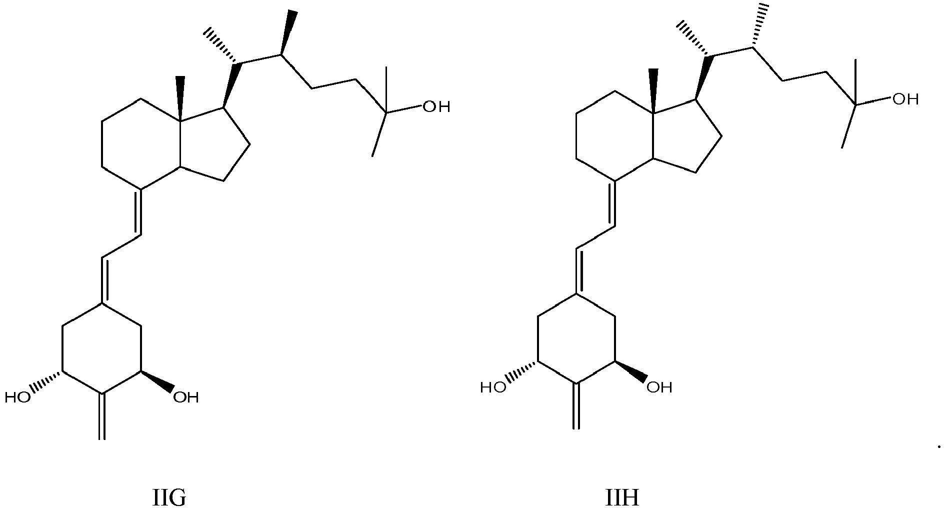

Preparation of (20S,22R)-2-methylene-19-nor-22-methyl-1α,25-dihydroxyvitamin D₃, (20S,22S)-2-methylene-19-nor-22-methyl-1α,25-dihydroxyvitamin D₃, comparative (20R,22S)-2-methylene-19-nor-22-methyl-1α,25-dihydroxyvitamin D₃, and comparative (20R,22R)-2-methylene-19-nor-22-methyl-1α,25-dihydroxyvitamin D₃ can be accomplished by condensing an appropriate bicyclic Windaus-Grundmann type ketone (IIIA, IIIB, IIIC, or IIID) with the allylic phosphine oxide IV followed by deprotection (removal of the Y₁ and Y₂ groups).

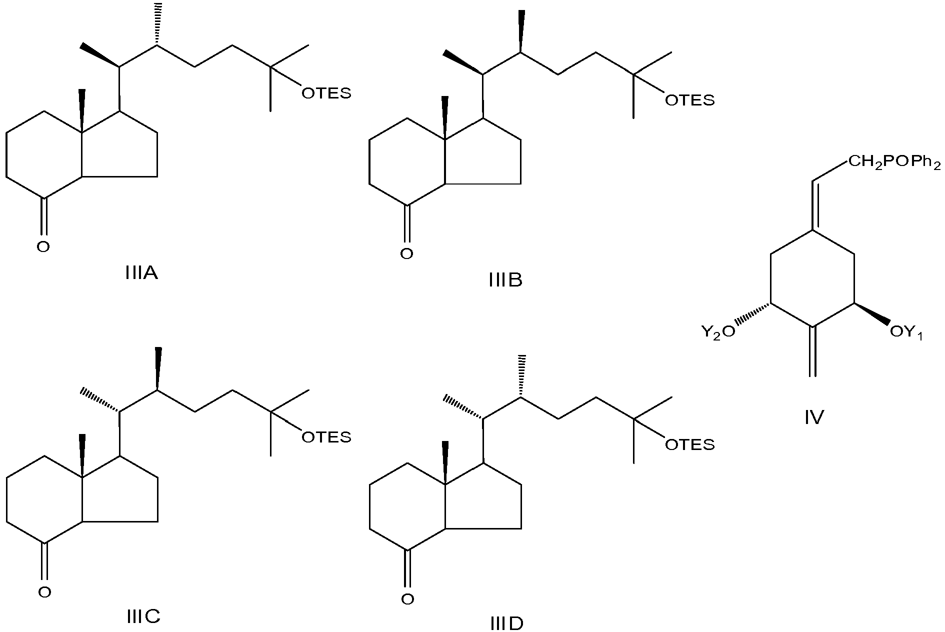

Hydraindanones of structure IIIA, IIIB, IIIC, or IIID can prepared by slight modification known methods as will be readily apparent to one of skill in the art and described herein. Specific examples of some important bicyclic ketones used to synthesize vitamin D analogs are those described in Mincione et al., Synth. Commun 19, 723, (1989); and Peterson et al., J. Org. Chem. 51, 1948, (1986). An overall process for synthesizing 2-alkylidene-19-nor-vitamin D compounds is illustrated and described in U.S. Patent No. 5,843,928. Details of preparing hydraindanones IIIA, IIIB, IIIC, and IIID are found in the Examples herein.

In phosphine oxide IV, Y₁ and Y₂ are hydroxy-protecting groups such as silyl protecting groups. The t-butyldimethylsilyl (TBDMS) group is an example of a particularly useful hydroxy-protecting group. The process described above represents an application of the convergent synthesis concept, which has been applied effectively to the preparation of numerous vitamin D compounds (see Lythgoe et al., J. Chem. Soc. Perkin Trans. I, 590 (1978); Lythgoe, Chem. Soc. Rev. 9, 449 (1983); Toh et al., J. Org. Chem. 48, 1414 (1983); Baggiolini et al., J. Org. Chem. 51, 3098 (1986); Sardina et al., J. Org. Chem. 51, 1264 (1986); J. Org. Chem. 51, 1269 (1986); DeLuca et al., U.S. Patent No. 5,086,191; DeLuca et al., U.S. Patent No. 5,536,713; and DeLuca et al., U.S. Patent No. 5,843,928).

Phosphine oxide IV is a convenient reagent that may be prepared according to the procedures described by Sicinski et al., J. Med. Chem., 41, 4662 (1998), DeLuca et al., U.S. Patent No. 5,843,928; Perlman et al., Tetrahedron Lett. 32, 7663 (1991); and DeLuca et al., U.S. Patent No. 5,086,191. Scheme 1 shows the general procedure for synthesizing phosphine oxide IV as outlined in U.S. Patent No. 5,843,928.

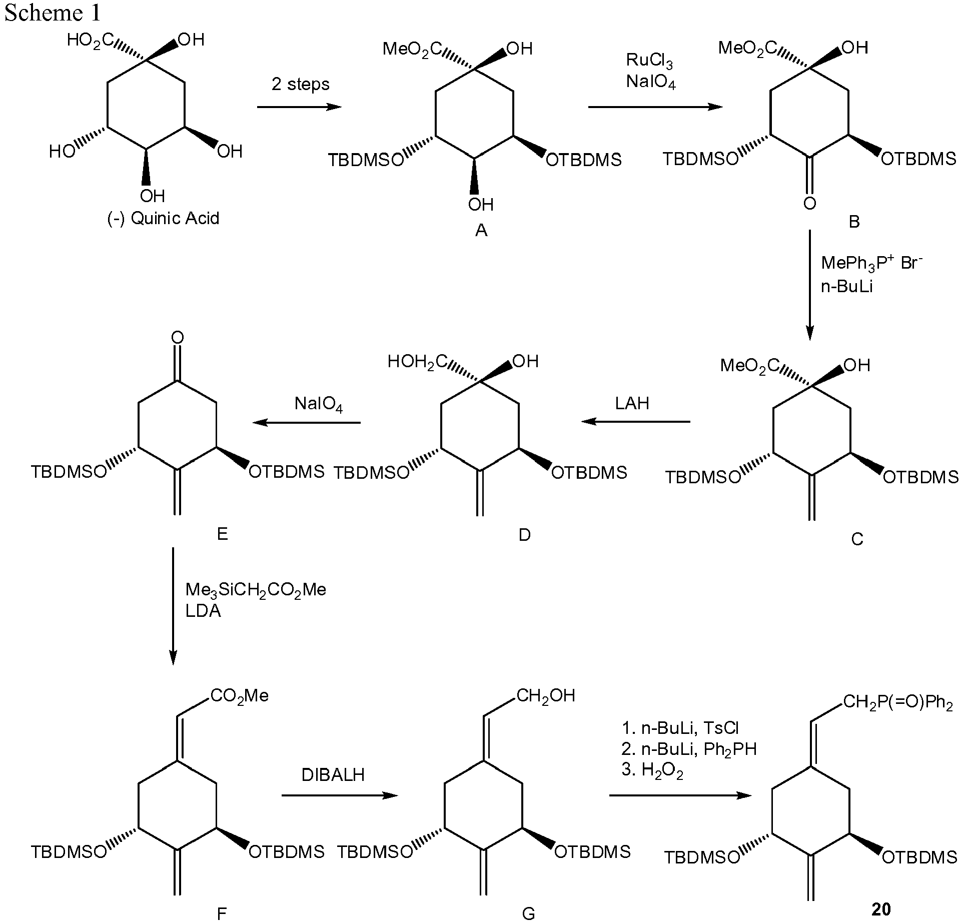

As used herein, the term "hydroxy-protecting group" signifies any group commonly used for the temporary protection of the hydroxy (-OH) functional group, such as, but not limited to, alkoxycarbonyl, acyl, alkylsilyl or alkylarylsilyl groups (hereinafter referred to simply as "silyl" groups), and alkoxyalkyl groups. Alkoxycarbonyl protecting groups are alkyl-O-CO- groups such as methoxycarbonyl, ethoxycarbonyl, propoxycarbonyl, isopropoxycarbonyl, butoxycarbonyl, isobutoxycarbonyl, tert-butoxycarbonyl, benzyloxycarbonyl or allyloxycarbonyl. The term "acyl" signifies an alkanoyl group of 1 to 6 carbons, in all of its isomeric forms, or a carboxyalkanoyl group of 1 to 6 carbons, such as an oxalyl, malonyl, succinyl, glutaryl group, or an aromatic acyl group such as benzoyl, or a halo, nitro or alkyl substituted benzoyl group. Alkoxyalkyl protecting groups are groups such as methoxymethyl, ethoxymethyl, methoxyethoxymethyl, or tetrahydrofuranyl and tetrahydropyranyl. Preferred silyl-protecting groups are trimethylsilyl, triethylsilyl, t-butyldimethylsilyl, dibutylmethylsilyl, diphenylmethylsilyl, phenyldimethylsilyl, diphenyl-t-butylsilyl and analogous alkylated silyl radicals. The term "aryl" specifies a phenyl-, or an alkyl-, nitro- or halo-substituted phenyl group. An extensive list of protecting groups for the hydroxy functionality may be found in Protective Groups in Organic Synthesis, Greene, T.W.; Wuts, P. G. M., John Wiley & Sons, New York, NY, (3rd Edition, 1999), which can be added or removed using the procedures set forth therein.

A "protected hydroxy" group is a hydroxy group derivatized or protected by any of the above groups commonly used for the temporary or permanent protection of hydroxy functional groups, *e.g.,* the silyl, alkoxyalkyl, acyl or alkoxycarbonyl groups, as previously defined.

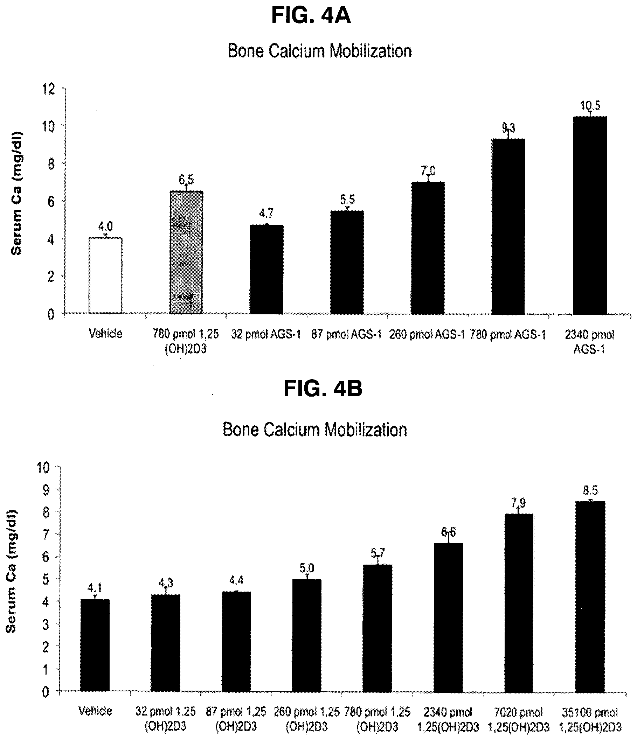

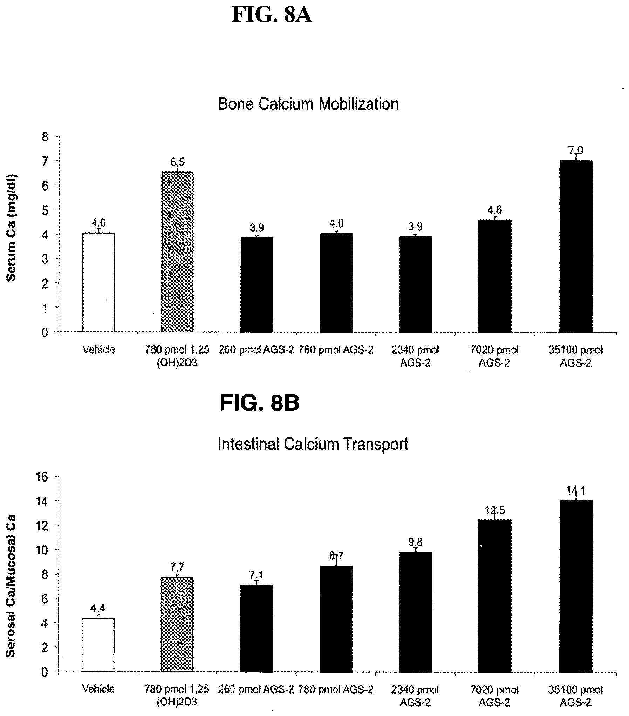

The compounds of the present technology show significant biological activity. AGS-1, AGS-2, comparative compound SAG-1, and comparative compound SAG-2 all bind the vitamin D receptor. In addition, both AGS-1, AGS-2, and comparative compound SAG-1 exhibit relatively high cell differentiation activity and AGS-1 and AGS-2 exhibit relatively high 24-hydroxylase transcription activity. The 24-hydroxylase transcription activity of comparative compound SAG-II was unexpectedly low in comparison to the native hormone, 1,25(OH)₂D₃ (Fig. 15). The calcemic activity profiles of the four compounds differ. AGS-1 displays significantly higher bone calcium mobilization activity and intestinal calcium transport activity than 1,25(OH)₂D₃ (See Figs. 4A-4C). By contrast, AGS-2 shows essentially no ability to mobilize bone calcium except at extremely high concentrations, but comparable or slightly higher intestinal calcium transport compared to 1,25(OH)₂D₃ (*See* Figs. 8A and 8B). Like, AGS-2, comparative compound SAG-1 shows little or no ability to mobilize bone calcium except at extremely high doses (*See* comparative Figs. 12A and 12B). However, in the case of intestinal calcium transport, comparative compound SAG-1 shows comparable or reduced potency in comparison to 1,25(OH)₂D₃ at lower concentrations but increased potency in comparison to 1,25(OH)₂D₃ at high concentrations (*See* comparative Figs. 12C and 12D). Comparative compound SAG-2, shows little or no ability to mobilize bone calcium, even at extremely high concentrations (*See* comparative Figs. 16A and 16B). In the case of intestinal calcium transport, comparative compound SAG-2 shows little ability to increase transport, except at extremely high concentrations.

In view of their biological activity, there is provided compounds of the present technology for use in the treatment and prophylaxis of human disorders which are characterized by an imbalance in the immune system, *e.g*., in autoimmune diseases, including multiple sclerosis, lupus, diabetes mellitus, host versus graft reaction, and rejection of organ transplants; and additionally for the treatment of inflammatory diseases, such as rheumatoid arthritis, asthma, and inflammatory bowel diseases such as celiac disease, ulcerative colitis and Crohn's disease. The compounds of the present technology may also be for use in the treatment of acne, alopecia and hypertension.

In view of the relatively high cell differentiation activity, there is also provided the present compounds for use in the treatment of psoriasis, or as anti-cancer agents, especially against leukemia, colon cancer, breast cancer and prostate cancer. In addition, due to their relatively high cell differentiation activity, these compounds provide a therapeutic agent for the treatment of various skin conditions including wrinkles, lack of adequate skin firmness, *i.e.,* slack skin. There is also provided the present compounds for use in the treatment of lack of adequate dermal hydration, i.e. dry skin and the treatment of insufficient sebum secretion. The compounds of the present teachings are thus not only for use in moisturizing of skin but also for use in improving the barrier function of skin.

In view of its extremely high cell differentiation activity and bone calcium mobilization activity, there is especially provided AGS-1 for use in the treatment of diseases such as psoriasis, osteoporosis, rickets, and renal osteodystrophy. In view of their cell differentiation and intestinal activities, AGS-2 is especially suited for use in the treatment of intestinal diseases such as IBD, including celiac disease and Crohn's disease.

The compounds of the present technology may be used to prepare pharmaceutical formulations or medicaments that include a compound of the present technology in combination with a pharmaceutically acceptable carrier. There is also provided such pharmaceutical formulations and medicaments for use in the treatment of various biological disorders such as those described herein. The compounds for use in treating such disorders typically include administering an effective amount of the compound or an appropriate amount of a pharmaceutical formulation or a medicament that includes the compound to a subject suffering from the biological disorder. In some embodiments, the subject is a mammal. In some such embodiments, the mammal is selected from a rodent, a primate, a bovine, an equine, a canine, a feline, an ursine, a porcine, a rabbit, or a guinea pig. In some such embodiments, the mammal is a rat or is a mouse. In some embodiments, the subject is a primate such as, in some embodiments, a human.

For treatment purposes, the compounds defined by formula IA, IB, IIA, IIB, IIE, and IIF, may be formulated for pharmaceutical applications as a solution in innocuous solvents, or as an emulsion, suspension or dispersion in suitable solvents or carriers, or as pills, tablets or capsules, together with solid carriers, according to conventional methods known in the art. Any such formulations may also contain other pharmaceutically acceptable and non-toxic excipients such as stabilizers, anti-oxidants, binders, coloring agents or emulsifying or taste-modifying agents. Pharmaceutically acceptable excipients and carriers are generally known to those skilled in the art and are thus included in the present technology. Such excipients and carriers are described, for example, in "Remingtons Pharmaceutical Sciences," Mack Pub. Co., New Jersey (1991).

The compounds may be administered orally, topically, parenterally, or transdermally. The compounds are advantageously administered by injection or by intravenous infusion or suitable sterile solutions, or in the form of liquid or solid doses via the alimentary canal, or in the form of creams, ointments, patches, or similar vehicles suitable for transdermal applications. In some embodiments, doses of from 0.001 µg to about 1 mg per day of the compound are appropriate for treatment purposes. In some such embodiments, an appropriate and effective dose may range from 0.01 µg to 1 mg per day of the compound. In other such embodiments, an appropriate and effective dose may range from 0.1 µg to 500 µg per day of the compound. Such doses will be adjusted according to the type of disease or condition to be treated, the severity of the disease or condition, and the response of the subject as is well understood in the art. The compound may be suitably administered alone, or together with another active vitamin D compound.

Compositions for use in the present technology include an effective amount of compound IA, IB, IIA, IIB, IIE, or IIF as the active ingredient, and a suitable carrier. An effective amount of the compound for use in accordance with some embodiments of the present technology will generally be a dosage amount such as those described herein, and may be administered topically, transdermally, orally, nasally, rectally, or parenterally.

The compound of formula IA, IB, IIA, IIB, IIE and IIF may be advantageously administered in amounts sufficient to effect the differentiation of promyelocytes to normal macrophages. Dosages as described above are suitable, it being understood that the amounts given are to be adjusted in accordance with the severity of the disease, and the condition and response of the subject as is well understood in the art.

The compound may be formulated as creams, lotions, ointments, aerosols, suppositories, topical patches, pills, capsules or tablets, or in liquid form as solutions, emulsions, dispersions, or suspensions in pharmaceutically innocuous and acceptable solvent or oils, and such preparations may contain, in addition, other pharmaceutically innocuous or beneficial components, such as stabilizers, antioxidants, emulsifiers, coloring agents, binders or taste-modifying agents.

The formulations of the present technology comprise an active ingredient in association with a pharmaceutically acceptable carrier and, optionally, other therapeutic ingredients. The carrier must be "acceptable" in the sense of being compatible with the other ingredients of the formulations and not deleterious to the recipient thereof.

Formulations of the present technology suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations for rectal administration may be in the form of a suppository incorporating the active ingredient and carrier such as cocoa butter, or in the form of an enema.

Formulations suitable for parenteral administration conveniently comprise a sterile oily or aqueous preparation of the active ingredient which is preferably isotonic with the blood of the recipient.

Formulations suitable for topical administration include liquid or semi-liquid preparations such as liniments, lotions, applicants, oil-in-water or water-in-oil emulsions such as creams, ointments or pastes; or solutions or suspensions such as drops; or as sprays.

For nasal administration, inhalation of powder, self-propelling or spray formulations, dispensed with a spray can, a nebulizer or an atomizer can be used. The formulations, when dispensed, preferably have a particle size in the range of 10 to 100 microns.

The formulations may conveniently be presented in dosage unit form and may be prepared by any of the methods well known in the art of pharmacy. By the term "dosage unit" is meant a unitary, *i.e*., a single dose which is capable of being administered to a patient as a physically and chemically stable unit dose comprising either the active ingredient as such or a mixture of it with solid or liquid pharmaceutical diluents or carriers.

The present technology is further illustrated by the following examples.

### EXAMPLES

### Example 1A: Synthesis of (20S,22S)-2-methylene-19-nor-22-methyl-1α,25-dihydroxyvitamin D₃ and (20S,22R)-2-methylene-19-nor-22-methyl-1α,25-dihydroxyvitamin D₃

Compounds of formula I, formula II, formula IIA and formula IIB were prepared using the methods shown in Schemes 2 and 3. As shown in Scheme 2, compound **2** was obtained by ozonolysis of vitamin D₂ (1) as described by Sicinski et al. (J. Med. Chem. 41, 4662-4672, 1998), followed by reduction with borohydride. Treatment of the dialcohol **2** with tosyl chloride in pyridine provided the tosyl protected compound **3.** Compound **3** was reacted with triethylsilyl trifluoromethanesulfonate and 2,6-lutidine in dichloromethane to yield compound **4.** Compound **4** was treated with sodium bicarbonate in DMSO to oxidize the tosyl protected alcohol group to an aldehyde compound **5.** Compound **5** was racemized at position 20 by treatment with tetrabutylammonium hydroxide and the resulting compound **6** was reduced with sodium borohydride to give pure isomer **7** along with a mixture of both isomers **7** and **8.** The isolated isomer **7** was then protected with tosyl chloride in pyridine and the tosyl protected alcohol **9** was converted to cyanide **10** by reacting it with sodium cyanide in DMSO. The cyano compound **10** was then treated with 4-bromo-2-methyl-1-triethylsilyloxy butane **(11),** in presence of a mixture of n-butyllithium and diisopropylamine, to provide compound **12.** The cyano group of compound **12** was converted to the corresponding aldehyde **13** by treating it with diisobutylaluminum hydride in dichloromethane. Aldehyde **13** was then reduced to alcohol **14** using sodium borohydride in methanol. The free hydroxyl group of compound **14** was then reacted with tosyl chloride in pyridine and the resulting tosyl protected compound **15** was reduced to the corresponding alkane **16** using lithium aluminum hydride as the reducing agent. The triethylsilyl protected dihydroxy compound 16 was then deprotected using tetrabutylammonium fluoride in THF and the racemic mixture of diols thus obtained was separated by crystallization from ethyl acetate to provide the two separate isomers, the 22*R* **17** diol and 22S diol **18**. Each of the diols **17** and **18** were then separately oxidized with a using tetrapropylammonium perruthenate in the presence of 4-methylmorpholine oxide to produce the respective ketones. Each ketone was further independently treated with triethylsilyl trifluoromethanesulfonate and 2,6-lutidine in dichloromethane to provide the triethylsilyl protected ketone 22R compound **19A** or 22S compound **19B.** 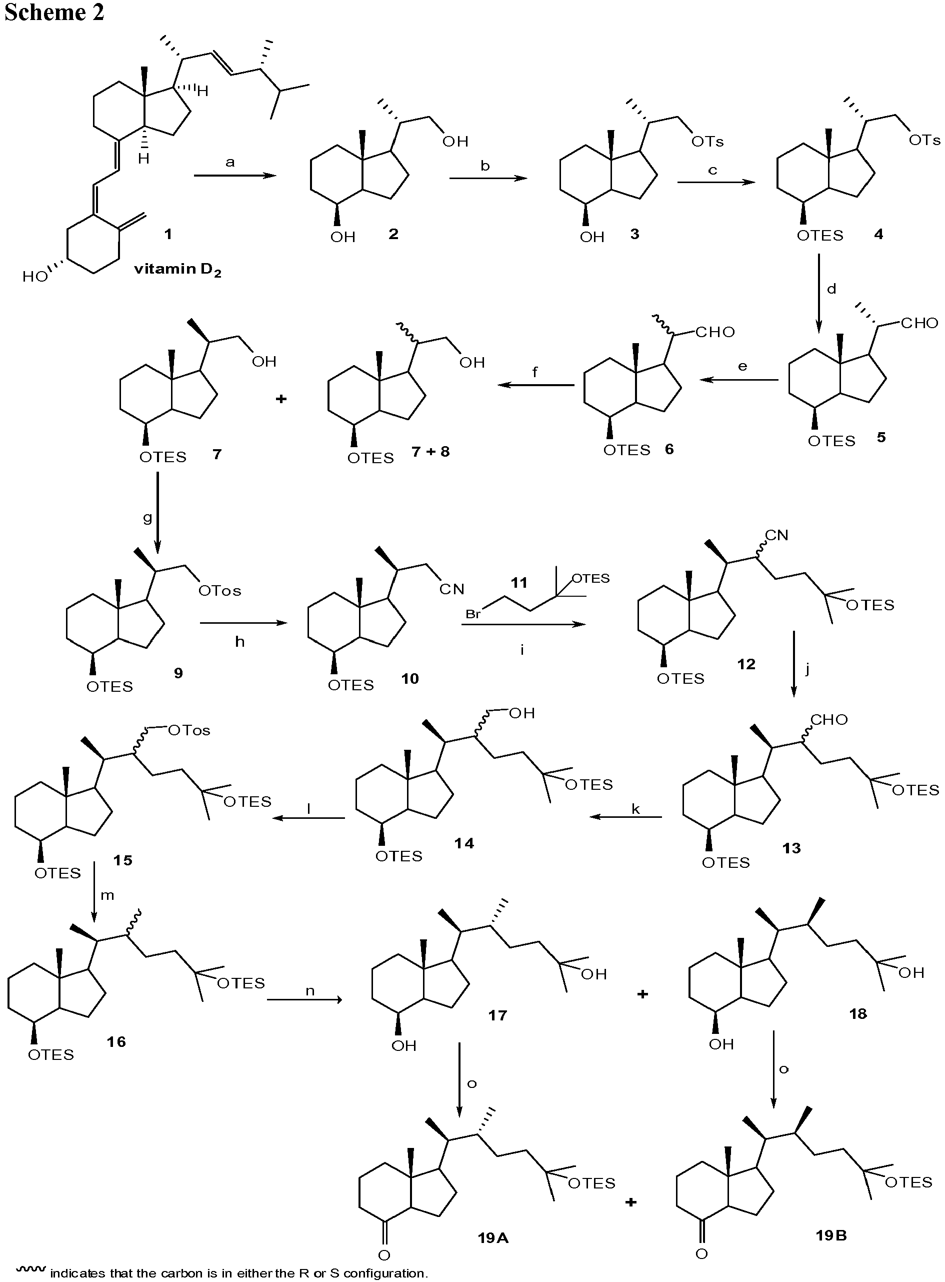
a) 1. O₃, pyridine, MeOH; 2. NaBH₄ (**2**, 49%)
b) TsCl, pyridine (**3**, 96%)
c) TESOTf, 2,6-lutidine, DCM (**4**, 99%)
d) NaHCO₃, DMSO, EtOAc (**5**, 76%)
e) 1. TBAOH, H₂O, DCM (**6**, 71%)
f) NaBH₄, THF, EtOH (**7**, 47%)(**8**, 40%)
g) TosCl, pyridine (**9**, 89%)
h) NaCN, DMSO (**10**, 85%)
i) 1. n-BuLi, DIPA, THF; 2. **11** (**12**, 79%)
j) DIBAL, toluene, DCM (**13**, 76%)
k) NaBH₄, MeOH (**14**, 70%)
l) TosCl, pyridine (**15**, 83%)
m) LiAlH₄, DEE (**16**, 75%)
n) 1. TBAF, THF (**17** and **18**, 99%); 2. Crystallization from EtOAc
o) 1. Mol sieves 4Å, 4-MMO, TPAP, DCM; 2. TESOTf, 2,6-lutidine, DCM (**19A**, 68%) **(19B,** 73%)

Scheme 3 illustrates the conversion of compounds **19A** or **19B** to the title compounds IIA or IIB. A Wittig-Horner condensation of the protected Grundmann's Ketone (Compound **19A** or **19B**) with the phosphine oxide (Compound **20)** in the presence of phenyllithium was performed as shown is Scheme 3. The Ring-A phosphine oxide compound **20** was synthesized as shown in Scheme 1 and as previously described. Finally, the target compound (Compound **IIA** or **IIB**) was generated by deprotection of hydroxy groups in compounds **21A** or **21B** in the presence of hydrofluoric acid.

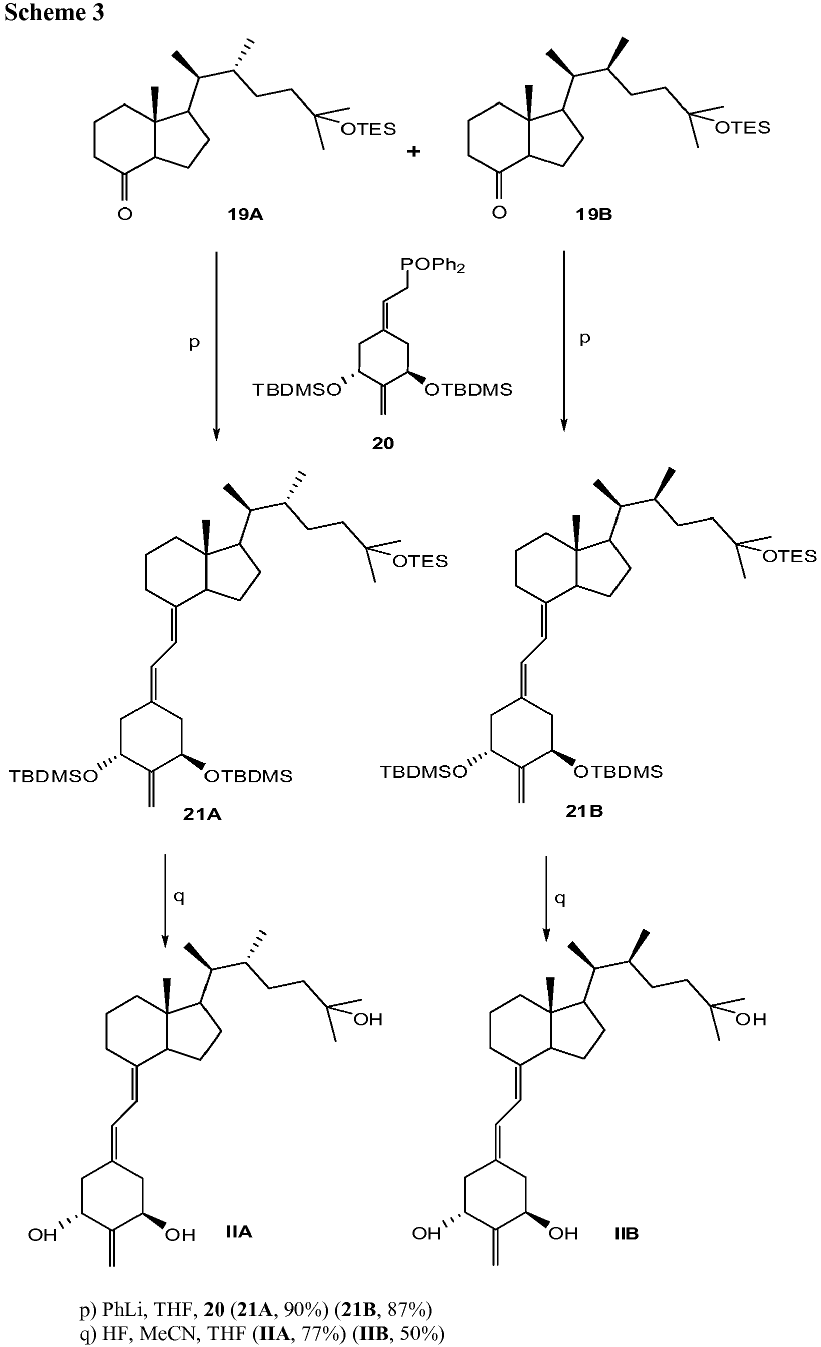

### (8S,20S)-Des-A,B-20-(hydroxymethyl)-pregnan-8-ol (2)

Ozone was passed through a solution of vitamin D₂ 1 (5 g, 12.6 mmol) and pyridine (5 mL, 4.89 g, 62 mmol) in methanol (400 mL) at -78 °C. When the reaction mixture turned deep blue it was flushed with oxygen for 15 min to remove the residual ozone and then it was treated with NaBH₄ (1.5 g, 40 mmol). After 15 min the second portion of NaBH₄ (1.5 g, 40 mmol) was added and the mixture was allowed to warm to room temperature. The third portion of NaBH₄ (1.5 g, 40 mmol) was added and the reaction mixture was stirred for 18 hours. The reaction was quenched with water, concentrated under reduced pressure and extracted with dichloromethane. The combined organic phases were washed with 1M aqueous HCl, saturated aqueous NaHCO₃ and dried (Na₂SO₄) and concentrated. The residue was purified by column chromatography on silica gel (30%, then 50% ethyl acetate/hexane) to give the diol **2** (2.61 g, 49%) as colorless crystals.
m.p. 107 °C (from ethyl acetate/hexane); [α]_{D}+32.9 (c 1.0, CHCl₃); ¹H NMR (500 MHz, CDCl₃) δ 4.07 (1H, d, J = 2.5 Hz), 3.62 (1H, dd, J = 10.5, 3.2 Hz), 3.37 (1H, dd, J = 10.5, 6.8 Hz), 1.98 (1H, m), 1.80 (3H, m), 1.02 (3H, d, J = 6.6 Hz), 0.94 (3H, s); ¹³C NMR (125 MHz, CDCl₃) δ 69.21, 67.81, 52.91, 52.34, 41.84, 40.20, 38.22, 33.55, 26.64, 22.55, 17.38, 16.60, 13.56; MS (EI) *m*/*z* 212 (1, M⁺), 194 (28, M⁺- H₂O), 179 (29), 163 (22), 147 (15), 135 (42), 125 (48), 111 (100), 97 (51); exact mass calculated for C₁₃H₂₂O (M⁺-H₂O) 194.1671, found 194.1673.

### (8S,20S)-Des-A,B-20-[(p-toluenesulfonyl)oxylmethyl-pregnan-8-ol (3)

A precooled (-20 °C) solution of tosyl chloride (0.9 g, 4.73 mmol) in pyridine (2 mL) was added to a mixture of the diol **2** (0.52 g, 2.45 mmol) in dry pyridine (5 mL) at -20 °C. The reaction mixture was stirred for 3 h at -20 °C, then it was warmed to 0 °C and stirred for 18 h. The mixture was pulled into a saturated aqueous CuSO₄ solution and extracted with dichloromethane. Combined organic phases were washed with a saturated aqueous CuSO₄ solution and dried (Na₂SO₄) and concentrated. The residue was purified by column chromatography on silica gel (20% ethyl acetate/hexane) to afford of tosylate **3** (0.86 g, 96% yield) as colorless crystals.
m.p. 95 °C (from ethyl acetate/hexane); [α]_{D}+17.4 (*c* 1.0, CHCl₃); ¹H NMR (400 MHz, CDCl₃) δ 7.77 (2H, d, J = 8.2 Hz), 7.34 (2H, d, J = 8.2 Hz), 4.06 (1H, s), 3.94 (1H, dd, J = 9.2, 3.1 Hz), 3.80 (1H, dd, J = 9.2, 6.2 Hz), 2.44 (3H, s), 1.90 (1H, m), 1.78 (2H, m), 0.95 (3H, d, J = 6.6 Hz), 0.88 (3H, s); ¹³C NMR (100 MHz, CDCl₃) δ 144.59, 133.01, 129.73, 127.86, 75.56, 68.98, 52.18, 41.81, 40.00, 35.66, 33.50, 26.36, 22.40, 21.60, 17.29, 16.69, 13.43; MS (EI) *m*/*z* 367 (6, MH⁺), 348 (5, M⁺ - H₂O), 307 (2), 194 (18), 179 (23), 150 (17), 135 (16), 125 (34), 111 (100), 91 (50); MS (ESI) *m*/*z* 389 (100, [M+Na]⁺), 755 (90, [2M+Na]⁺), 1121 (60, [3M+Na]⁺); exact mass calculated for C₂₀H₃₀O₄SNa [M+Na]⁺ 389.1763, found 389.1758.

### (8S,20S)-Des-A,B-8-[(triethylsilyl)oxy]-20-[(p-toluenesulfonyl)oxy]methyl-pregnane (4)

Triethylsilyl trifluoromethanesulfonate (0.6 mL, 0.70 g, 2.65 mmol) was added to a solution of the tosylate **3** (0.65 g, 1.78 mmol) and 2,6-lutidine (0.3 mL, 0.28 g, 2.58 mmol) in dichloromethane (6 mL) at 0 °C. The reaction mixture was stirred for 15 min and it was diluted with dichloromethane. The organic phase was washed with water, dried (Na₂SO₄) and concentrated. The residue was purified by column chromatography on silica gel (20% ethyl acetate/hexane) to give the product **4** (0.84 g, 99% yield) as a light yellow oil.
[α]_{D} +20.6 (c 1.0, CHCl₃); ¹H NMR (400 MHz, CDCl₃) δ 7.78 (2H, d, J = 8.2 Hz), 7.34 (2H, d, J = 8.2 Hz), 4.01 (1H, d, J = 2.0 Hz), 3.96 (1H, dd, J = 9.2, 3.0 Hz), 3.79 (1H, dd, J = 9.2, 6.5 Hz), 2.45 (3H, s), 1.87 (1H, m), 0.94 (3H, d, J = 5.9 Hz), 0.93 (9H, t, J = 7.9 Hz), 0.86 (3H, s), 0.54 (6H, q, J = 7.9 Hz); ¹³C NMR (125 MHz, CDCl₃) δ 144.55 (0), 133.10 (0), 129.73 (1), 127.91 (1), 75.76 (2), 69.11 (1), 52.70 (1), 52.36 (1), 42.12 (0), 40.39 (2), 35.72 (1), 34.47 (2), 26.52 (2), 22.88 (2), 21.63 (3), 17.56 (2), 16.76 (3), 13.46 (3), 6.91 (3), 4.89 (2); MS (EI) *m*/*z* no M⁺, 319 (46), 291 (9), 265 (9), 246 (5), 217 (100), 189 (81), 161 (69), 133 (54), 103 (38), 94 (39); MS (ESI) *m*/*z* 503 (100, [M+Na]⁺), 983 (40, [2M+Na]⁺), 1463 (71, [3M+Na]⁺); exact mass calculated for C₂₆H₄₄O₄SSiNa [M+Na]⁺ 503.2627, found 503.2629.

### (8S,20S)-Des-A,B-8-[(triethylsilyl)oxy]-20-(formyl)-pregnane (5)

Sodium bicarbonate (5 g, 59.5 mmol) was added to a solution of tosylate **4** (2.31g, 4.81 mmol) in DMSO (15 mL). The reaction mixture was stirred for 1 hour 15 min at 120 °C and it was diluted with ethyl acetate. The organic phase was washed with brine, dried (Na₂SO₄) and concentrated. The residue was purified by column chromatography on silica gel (5% ethyl acetate/hexane) to give the product **5** (1.19 g, 76% yield) as a colorless oil.
[α]_{D} +41.4 (c 1.0, CHCl₃); ¹H NMR (400 MHz, CDCl₃) δ 9.58 (1H, d, J = 3.2 Hz), 4.06 (1H, d, J = 2.4 Hz), 2.36 (1H, m), 1.09 (3H, d, J = 6.8, 3.0 Hz), 0.96 (3H, s), 0.94 (9H, t, J = 7.9 Hz), 0.56 (6H, q, J = 7.9 Hz); ¹³C NMR (125 MHz, CDCl₃) δ 205.40 (1), 69.01 (1), 52.38 (1), 51.69 (1), 49.17 (1), 42.64 (0), 40.49 (2), 34.54 (2), 26.20 (2), 23.28 (2), 17.58 (2), 13.89 (3), 13.32 (3), 6.92 (3), 4.90 (2); MS (EI) *m*/*z* 324 (5, M⁺), 295 (100, M⁺- EtOH), 281 (30), 246 (12), 191 (36), 175 (99), 135 (54), 103 (76); MS (ESI) *m*/*z* 671 (100, [2M+Na]⁺), 995 (49, [3M+Na]⁺); exact mass calculated for C₁₇H₃₁O₂Si [M-Et]⁺ 295.2093, found 295.2103.

### (8S,20R)-Des-A,B-8-[(triethylsilyl)oxy]-20-(hydroxymethyl)-pregnane (7)

Tetrabutylammonium hydroxide (40 wt. % solution in water, 4 mL, 3.98 g, 0.015 mol) was added to a solution of aldehyde **5** (0.97 g, 2.99 mmol) in dichloromethane (20 mL). The reaction mixture was stirred for 18 hours at room temperature and it was diluted with dichloromethane. The organic phase was washed with water, dried (Na₂SO₄) and concentrated. The product was purified by column chromatography on silica gel (3%, then 5% ethyl acetate/hexane) to give a mixture of isomers **6** (0.69 g, 71 % yield). Sodium borohydride (0.2 g, 5.29 mmol) was added to a solution of aldehydes **6** (0.69 g, 2.13 mmol) in THF (10 mL) and ethanol (10 mL). The reaction mixture was stirred for 45 min, quenched with saturated NH₄Cl, extracted with ethyl acetate and dried (Na₂SO₄). The residue was purified by column chromatography on silica gel (4%, then 20% ethyl acetate/hexane) to give the pure isomer **7** (0.326 g, 47% yield) and a mixture of both isomers **7** and **8** (0.277 g, 40% yield).
[α]_{D} +33.6 (c 1.0, CHCl₃); ¹H NMR (500 MHz, CDCl₃) δ 4.03 (1H, d, J = 2.5 Hz), 3.72 (1H, dd, J = 10.7, 3.6 Hz), 3.44 (1H, dd, J = 10.7, 7.0 Hz), 0.95 (9H, t, J = 7.9 Hz), 0.94 (3H, d, J = 6.6 Hz), 0.93 (3H, s), 0.55 (6H, q, J = 7.9 Hz); ¹³C NMR (125 MHz, CDCl₃) δ 69.25 (1), 66.84 (2), 53.01 (1), 41.91 (0), 40.20 (2), 37.49 (1), 34.58 (2), 26.73 (2), 22.81 (2), 17.67 (2), 16.58 (3), 13.88 (3), 6.93 (3), 4.91 (2); MS (EI) *m*/*z* 326 (7, M⁺), 311 (3, M⁺-CH₃), 297 (100, M⁺-Et), 283 (41), 265 (8), 225 (23), 193 (41), 177 (41), 135 (57), 103 (99); MS (ESI) *m*/*z* 327 (100, [M+H]⁺); exact mass calculated for C₁₇H₃₃O₂Si [M-Et]⁺297.2250, found 297.2244.

### (8S,20R)-Des-A,B-8-[(triethylsilyl)oxyl-20-[(p-toluenesulfonyl)oxy]methyl-pregnane (9)

A solution of tosyl chloride (0.38 g, 2 mmol) in pyridine (3 mL) was transferred *via* cannula to a solution of alcohol 7 (0.326 g, 1 mmol) in pyridine (5 mL) at -20 °C. The reaction mixture was stirred at -20 °C for 1 hour and then at +4 °C overnight. It was diluted with methylene chloride, washed with a saturated aqueous solution of CuSO₄ and dried (Na₂SO₄). The residue was purified by column chromatography on silica gel (5%, then 10% and 20% ethyl acetate/hexane) to give the tosylate 9 (427 mg, 89% yield) as a colorless oil.
[α]_{D} +8.8 (c 1.0, CHCl₃); ¹H NMR (400 MHz, CDCl₃) δ 7.78 (1H, d, J = 8.2 Hz), 7.34 (1H, d, J = 8.2 Hz), 4.11 (1H, dd, J = 9.3, 3.4 Hz), 4.00 (1H, d, J = 2.0 Hz), 3.77 (1H, dd, J = 9.3, 7.4 Hz), 2.45 (3H, s), 0.93 (9H, t, J = 7.9 Hz), 0.87 (3H, d, J = 6.7 Hz), 0.81 (3H, s), 0.53 (6H, q, J = 7.9 Hz); ¹³C NMR (100 MHz, CDCl₃) δ 144.53 (0), 133.18 (0), 129.70 (1), 127.93 (1), 74.30 (2), 69.09 (1), 52.74 (1), 52.60 (1), 41.77 (0), 39.95 (2), 34.66 (1), 34.40 (2), 26.61 (2), 22.64 (2), 21.62 (3), 17.54 (2), 16.65 (3), 13.82 (3), 6.91 (3), 4.89 (2); MS (EI) *m*/*z* 480 (18, M⁺), 465 (2), 437 (14), 348 (2, M⁺- Et₃SiOH), 309 (1, M⁺- CH₃C₆H₄SO₃), 257 (91), 225 (23), 177 (100), 135 (19), 121 (24); MS (ESI) *m*/*z* 503 (7, [M+Na]⁺), 983 (4, [2M+Na]⁺), 1463 (10, [3M+Na]⁺); exact mass calculated for C₂₆H₄₄O₄SSiNa [M+Na]⁺ 503.2627, found 503.2639.

### (8S,20S)-Des-A,B-8-[(triethylsilyl)oxy]-20-(cyanomethyl)-pregnane (10)

Sodium cyanide (0.9 g, 18.4 mmol) was added to a solution of tosylate **9** (0.412 g, 0.858 mmol) in DMSO (5 mL). The resulting mixture was stirred at 90 °C for 2 h, then it was cooled, diluted with water and extracted with ethyl acetate. Combined organic phases were dried (Na₂SO₄) and concentrated. The residue was purified by column chromatography on silica gel (10% ethyl acetate/hexane) to give cyanide **10** (0.242 g, 85% yield) as a colorless oil.
[α]_{D} +17.3 (c 1.0, CHCl₃); ¹H NMR (400 MHz, CDCl₃) δ 4.04 (1H, d, J = 2.2 Hz), 2.44 (1H, dd, J = 16.7, 4.0 Hz), 2.38 (1H, dd, J = 16.7, 6.6 Hz), 1.06 (3H, d, J = 6.7 Hz), 0.94 (9H, t, J = 7.9 Hz), 0.91 (3H, s), 0.55 (6H, q, J = 7.9 Hz); ¹³C NMR (100 MHz, CDCl₃) δ 118.90 (0), 69.07 (1), 54.96 (1), 52.74 (1), 41.91 (0), 40.23 (2), 34.29 (2), 31.79 (1), 27.01 (2), 24.00 (2), 22.68 (2), 19.58 (3), 17.53 (2), 13.81 (3), 6.90 (3), 4.88 (2); MS (EI) *m*/*z* 335 (3, M⁺), 320 (1, M⁺ - Me) 306 (76, M⁺- Et), 292 (15), 271 (2), 225 (3), 202 (30), 161 (13), 103 (100), 75 (38); MS (ESI) *m*/*z* 336 (7, [M+H]⁺), 358 (4, [M+Na]⁺), 693 (100, [2M+Na]⁺), 1028 (40, [3M+Na]⁺); exact mass calculated for C₁₈H₃₂NOSi [M-Et]⁺306.2253, found 306.2253.

### (8S,20S,22ξ)-Des-A,B-8-[(triethylsilyl)oxy]-22-cyano-25-[(triethylsilyl)oxy]-cholestane (12)

n-Butyllithium (1.6 M in hexane, 1.2 mL, 0.123 g, 1.92 mmol) was added to a solution of diisopropylamine (0.26 mL, 0.186 g, 1.84 mmol) in THF (4 mL) at 0 °C. The resulting mixture was stirred at 0 °C for 30 min, then it was cooled to -78 °C and a solution of cyanide **10** (0.239 g, 0.713 mmol) in THF (3 mL) was added. The mixture was stirred at -78 °C for 30 min and a solution of bromide 11 (0.41 g, 1.46 mmol) was added. The reaction mixture was stirred at -78 °C for 1 h and then at 0 °C for 1 h. It was quenched with a saturated aqueous NH₄Cl solution and extracted with ethyl acetate. Combined organic phases were washed with brine, dried (Na₂SO₄) and concentrated. The residue was purified by column chromatography on silica gel (1%, then 10% ethyl acetate/hexane) to give a mixture of cyanides **12** (0.298 g, 79% yield).
Major isomer: ¹H NMR (400 MHz, CDCl₃) δ 4.04 (1H, s), 2.80 (1H, m), 1.22 (3H, s), 1.21 (3H, s), 0.97 (3H, d, J = 7.0 Hz), 0.94 (18H, t, J = 7.9 Hz), 0.90 (3H, s), 0.57 (6H, q, J = 7.9 Hz), 0.55 (6H, q, J = 7.9 Hz); ¹³C NMR (100 MHz, CDCl₃) δ 121.43 (0), 72.66 (0), 69.19 (1), 54.29 (1), 52.81 (1), 42.96 (2), 41.94 (0), 40.42 (2), 36.58 (1), 36.48 (1), 34.34 (2), 30.16 (3), 29.57 (3), 27.21 (2), 25.86 (2), 22.68 (2), 17.59 (2), 14.37 (3), 13.78 (3), 7.08 (3), 6.92 (3), 6.70 (2), 4.90 (2); MS (EI) *m*/*z* no M⁺, 491 (3), 476 (100), 345 (6), 280 (16), 246 (5), 216 (3), 189 (8), 155 (7), 132 (22), 91 (24); exact mass calculated for C₂₉H₅₆NO₂Si₂ [M-Et]⁺ 506.3850, found 506.3848.

### (8S,20S,22ξ)-Des-A,B-8-[(triethylsilyl)oxy]-22-formyl-25-[(triethylsilyl)oxy]-cholestane (13)

Diisobutylaluminum hydride (1.5 M in toluene, 0.56 mL, 0.119 g, 0.84 mmol) was added to a solution of cyanides **12** (0.3 g, 0.56 mmol) in dichloromethane (4 mL) at -10 °C. The reaction mixture was stirred at -10 °C for 1 hour, then it was quenched with a saturated aqueous sodium potassium tartrate solution (5 mL). The water phase was extracted with dichloromethane. Combined organic layers were washed with brine and dried (Na₂SO₄) and concentrated. The residue was purified by column chromatography on silica gel (3% ethyl acetate/hexane) to give a mixture of aldehydes **13** (0.228 g, 76% yield).
Major isomer: ¹H NMR (400 MHz, CDCl₃) δ 9.78 (1H, d, J = 2.4 Hz), 4.04 (1H, d, J = 1.8 Hz), 2.52 (1H, m), 1.21 (3H, s), 1.20 (3H, s), 0.95 (3H, d, J = 8.0 Hz) covered by 0.95 (9H, t, J = 7.9 Hz), 0.94 (9H, t, J = 7.9 Hz), 0.92 (3H, s), 0.56 (6H, q, J = 7.9 Hz), 0.55 (6H, q, J = 7.9 Hz); ¹³C NMR (100 MHz, CDCl₃) δ 206.75 (1), 73.08 (0), 69.23 (1), 54.52 (1), 53.87 (1), 52.86 (1), 42.95 (2), 42.53 (0), 40.63 (2), 36.04 (1), 34.53 (2), 30.07 (3), 29.56 (3), 27.02 (2), 22.79 (2), 22.08 (2), 17.67 (2), 14.40 (3), 14.07 (3), 7.11 (3), 6.94 (3), 6.75 (2), 4.92 (2); MS (ESI) *m*/*z* 539 (100, [M+H]⁺), 561 (70, [M+Na]⁺), 1099 (57, [2M+Na]⁺); exact mass calculated for C₃₁H₆₂O₃Si₂H [M+H]⁺ 539.4316, found 539.4312.

### (8S,20S,22ξ)-Des-A,B-8-[(triethylsilyl)oxy]-22-(hydroxymethyl)-25-[(triethylsilyl)oxy]-cholestane (14)

Sodium borohydride (0.2 g, 5.29 mmol) was added to a solution of aldehydes **13** (0.23 g, 0.427 mmol) in methanol (4 mL) at 0 °C. The reaction mixture was warmed to room temperature and stirred for 2 h, then it was quenched with water and extracted with ethyl acetate. Combined organic layers were washed with brine and dried (Na₂SO₄) and concentrated. The residue was purified by column chromatography on silica gel (3%, then 10% ethyl acetate/hexane) to give a mixture of alcohols **14** (0.16 g, 70% yield) as a colorless oil.
Major isomer: ¹H NMR (500 MHz, CDCl₃) δ 4.03 (1H, d, J = 2.2 Hz), 3.75 (1H, dd, J = 10.5, 3.9 Hz), 3.41 (1H, dd, J = 10.5, 8.5 Hz), 1.96 (1H, m), 1.210 (3H, s), 1.206 (3H, s), 0.95 (18H, t, J = 7.9 Hz), 0.92 (3H, s), 0.73 (3H, d, J = 7.0 Hz), 0.57 (6H, q, J = 7.9 Hz), 0.55 (6H, q, J = 7.9 Hz); ¹³C NMR (125 MHz, CDCl₃) δ 73.54 (0), 69.35 (1), 63.76 (2), 53.51 (1), 53.11 (1), 43.39 (1), 43.03 (2), 42.41 (0), 40.38 (2), 35.32 (1), 34.68 (2), 29.89 (3), 29.79 (3), 27.43 (2), 24.41 (2), 22.93 (2), 17.70 (2), 13.60 (3, C-18 and C-21), 7.12 (3), 6.94 (3), 6.77 (2), 4.94 (2);
Minor isomer (visible signals): ¹H NMR (500 MHz, CDCl₃) δ 3.61 (1H, dd, J = 10.9, 4.6 Hz), 3.47 (1H, dd, J = 10.9, 8.8 Hz); MS (ESI) *m*/*z* 541 (29, [M+H]⁺), 563 (100, [M+Na]⁺), 1103 (14, [2M+Na]⁺); exact mass calculated for C₃₁H₆₄O₃Si₂Na [M+Na]⁺ 563.4292, found 563.4313.

### (8S,20S,22ξ)-Des-A,B-8-[(triethylsilyl)oxy]-22-[(p-toluenesulfonyl)oxy]methyl-25-[(triethylsilyl)oxy]-cholestane (15)

A solution of tosyl chloride (0.3 g, 1.57 mmol) in pyridine (1 mL) was added to a mixture of alcohols **14** (0.16 g, 0.3 mmol) in dry pyridine (3 mL) at -20 °C. The reaction mixture was stirred at -20 °C for 1 hour and at +4 °C for 18 h. Then it was quenched with a saturated aqueous CuSO₄ solution and extracted with dichloromethane. Combined organic phases were dried (Na₂SO₄) and concentrated. The residue was purified by column chromatography on silica gel (3%, then 5% ethyl acetate/hexane) to give a mixture of tosylates **15** (0.17 g, 83% yield).
Major isomer: ¹H NMR (400 MHz, CDCl₃) δ 7.79 (2H, d, J = 8.2 Hz), 7.34 (2H, d, J = 8.1 Hz), 4.06 (1H, dd, J = 9.0, 3.8 Hz), 3.99 (1H, d, J = 2.0 Hz), 3.80 (1H, t, J = 9.0 Hz), 2.44 (3H, s), 1.16 (3H,s), 1.14 (3H, s), 0.93 (9H, t, J = 7.8 Hz), 0.92 (9H, t, J = 7.8 Hz), 0.85 (3H, s), 0.66 (3H, d, J = 7.0 Hz), 0.54 (12H, q, J = 7.8 Hz); MS (ESI) *m*/*z* 717 (15, [M+Na]⁺); exact mass calculated for C₃₈H₇₀O₅SSi₂Na [M+Na]⁺ 717.4380, found 717.4363.

### (8S,20S,22ξ)-Des-A,B-8-[(triethylsilyl)oxy]-22-methyl-25-[(triethylsilyl)oxy]-cholestane (16)

LiAlH₄ (0.2 g, 5.26 mmol) was added to a solution of tosylates **15** (0.17 g, 0.24 mmol) in dry diethyl ether (5 mL) at 0 °C. The reaction mixture was stirred at +4 °C for 20 h. The excess of LiAlH₄ was decomposed with water. The reaction mixture was diluted with diethyl ether and then it was filtered through Celite. The filtrate was extracted with ethyl acetate, dried (Na₂SO₄) and concentrated. The residue was purified by column chromatography on silica gel (3%, then 5% ethyl acetate/hexane) to give a mixture of products **16** (96 mg, 75% yield).
Major isomer: ¹H NMR (400 MHz, CDCl₃) δ 4.03 (1H, d, J = 1.7 Hz), 1.93 (1H, m), 1.18 (6H, s), 0.95 (18H, t, J = 7.9 Hz), 0.90 (3H, s), 0.73 (3H, d, J = 6.7 Hz), 0.67 (3H, d, J = 6.8 Hz), 0.56 (6H, q, J = 7.9 Hz), 0.55 (6H, q, J = 7.8 Hz); ¹³C NMR (100 MHz, CDCl₃) δ 73.48 (0), 69.47 (1), 53.62 (1), 53.23 (1), 43.29 (2), 42.25 (0), 40.39 (2), 38.10 (1), 34.74 (1 and 2), 30.31 (2), 29.89 (3, C-26 and C-27), 27.57 (2), 22.91 (2), 17.78 (2), 13.93 (3), 13.50 (3), 12.14 (3), 7.13 (3), 6.95 (3), 6.82 (2), 4.95 (2); MS (EI) *m*/*z* no M⁺, 506 (0.9, M⁺ - H₂O), 495 (46, M⁺ - Et), 481 (6), 391 (7), 363 (43), 349 (2), 307 (2), 259 (20), 245 (7), 225 (14), 173 (91), 135 (41), 103 (100); exact mass calculated for C₂₉H₅₉O₂Si₂ [M-Et]⁺ 495.4054, found 495.4058.

### (8S,20S,22R)-Des-A,B-22-methyl-cholestan-8,25-diol (17) and (8S,20S,22S)-Des-A,B-22-methyl-cholestan-8,25-diol (18)

Tetrabutylammonium fluoride (1.0 M in THF, 1 mL, 1 mmol) was added to a solution of compounds **16** (96.4 mg, 0.184 mmol) in THF (3 mL) at 0 °C. The reaction mixture was stirred at +4 °C for 20 h, then it was diluted with water and extracted with ethyl acetate. Combined organic extracts were dried (Na₂SO₄) and concentrated. The residue was purified by column chromatography on silica gel (30% ethyl acetate/hexane) to give a mixture of diols **17** and **18** (55 mg, 99% yield) in 2:1 ratio, respectively (based on ¹H NMR). Isomers were separated by crystallization from ethyl acetate and absolute configuration was established by X-ray analysis. Pure crystals (38.9 mg) of the isomer **17** were obtained after two crystallizations and the 22R absolute configuration of the diol **17** was established. Diol **18** (22*S*) (16.4 mg) containing a small amount of isomer 22R was obtained from the filtrate after second crystallization.
**17:** m.p. 133-134 °C (EtOAc); [α]_{D} +32.5 (c 1.0, CHCl₃); ¹H NMR (500 MHz, CDCl₃) δ 4.07 (1H, d, J = 1.9 Hz), 1.95 (1H, m), 1.21 (6H, s), 0.93 (3H, s), 0.76 (3H, d, J = 6.8 Hz), 0.69 (3H, d, J = 6.8 Hz); ¹³C NMR (125 MHz, CDCl₃) δ 71.08 (0), 69.41 (1), 53.42 (1), 52.70 (1), 42.13 (2), 41.95 (0), 39.97 (2), 38.04 (1), 34.65 (1), 33.59 (2), 30.27 (2), 29.30 (3), 29.15 (3), 27.42 (2), 22.36 (2), 17.49 (2), 13.80 (3), 13.52 (3), 12.06 (3); MS (EI) *m*/*z* no M⁺, 278 (46, M⁺ - H₂O), 260 (32, M⁺ - 2H₂O), 245 (16), 217 (9), 179 (20), 163 (47), 151 (48), 145 (63), 125 (69), 111 (100); MS (ESI) *m*/*z* 319 (18, [M+Na]⁺); exact mass calculated for C₁₉H₃₆O₂Na [M+Na]⁺ 319.2613, found 319.2623.
**18:** ¹H NMR (500 MHz, CDCl₃) δ 4.08 (1H, s), 1.93 (1H, m), 1.21 (6H, s), 0.92 (3H, s), 0.86 (3H,d, J = 6.8 Hz), 0.74 (3H, d, J = 6.8 Hz); ¹³C NMR (125 MHz, CDCl₃) δ 71.28 (0), 69.40 (1), 53.03 (1), 52.56 (1), 42.34 (2), 41.91 (0), 40.49 (1), 39.83 (2), 34.99 (1), 33.54 (2), 29.21 (3), 29.12 (3), 27.05 (2), 24.62 (2), 22.46 (2), 18.35 (3), 17.49 (2), 13.60 (3), 13.07 (3); MS (EI) *m*/*z* 296 (15, M⁺), 278 (33, M⁺ - H₂O), 260 (15, M⁺ - 2H₂O), 246 (100), 210 (6), 196 (18), 181 (36), 163 (29), 125 (28), 111 (65); exact mass calculated for C₁₉H₃₆O₂Na [M+Na]⁺ 319.2613, found 319.2605.

### (20S,22R)-Des-A,B-22-methyl-25-[(triethylsilyl)oxy]-cholestan-8-one (19A)

Molecular sieves 4Å (60 mg) were added to a solution of 4-methylmorpholine oxide (36 mg, 0.307 mmol) in dichloromethane (0.5 mL). The mixture was stirred at room temperature for 15 min and tetrapropylammonium perruthenate (3 mg, 8.54 µmol) was added, followed by a solution of diol **17** (15 mg, 0.051 mmol) in dichloromethane (400 + 300 µL). The resulting suspension was stirred at room temperature for 1 h. The reaction mixture was filtered through a Waters silica Sep-Pak cartridge (2 g) that was further washed with ethyl acetate. After removal of the solvent the ketone (15 mg) was obtained as a colorless oil.

Triethylsilyl trifluoromethanesulfonate (60µL, 70 mg, 0.265 mmol) was added dropwise to a solution of the ketone (15 mg, 0.051 mmol) and 2,6-lutidine (110 µL, 0.101 g, 0.94 mmol) in dichloromethane (2 mL) at -40 °C. The reaction mixture was stirred at -40 °C for 15 min, then it was diluted with dichloromethane and washed with water. The organic layer was dried (Na₂SO₄) and concentrated. The residue was applied to a Waters silica Sep-Pak cartridge (5 g). Elution with hexane/ethyl acetate (0.5% then 1%) gave the protected ketone **19A** (14 mg, 68% yield).

### (20S,22S)-Des-A,B-22-methyl-25-[(triethylsilyl)oxy]-cholestan-8-one (19B)

Molecular sieves 4Å (60 mg) were added to a solution of 4-methylmorpholine oxide (51 mg, 0.435 mmol) in dichloromethane (0.5 mL). The mixture was stirred at room temperature for 15 min and tetrapropylammonium perruthenate (7 mg, 0.02 mmol) was added, followed by a solution of diol **18** (14.3 mg, 0.048 mmol) in dichloromethane (400 + 300 µL). The resulting suspension was stirred at room temperature for 1 h. The reaction mixture was filtered through a Waters silica Sep-Pak cartridge (2 g) that was further washed with ethyl acetate. After removal of the solvent the ketone (15 mg) was obtained as a colorless oil.
Triethylsilyl trifluoromethanesulfonate (40µL, 46 mg, 0.176 mmol) was added dropwise to a solution of the ketone (15 mg, 0.051 mmol) and 2,6-lutidine (80 µL, 74 mg, 0.69 mmol) in dichloromethane (2 mL) at -40 °C. The reaction mixture was stirred at -40 °C for 15 min, then it was diluted with dichloromethane and washed with water. The organic layer was dried (Na₂SO₄) and concentrated. The residue was applied to a Waters silica Sep-Pak cartridge (5 g). Elution with hexane/ethyl acetate (1%) gave the protected ketone **19B** (14.4 mg, 73% yield).
¹H NMR (500 MHz, CDCl₃) δ 2.45 (1H, dd, J = 11.5, 7.2 Hz), 1.207 (3H, s), 1.205 (3H, s), 0.96 (9H, t, J = 8.0 Hz), 0.85 (3H, d, J = 6.8 Hz), 0.76 (3H, d, J = 6.9 Hz), 0.62 (3H, s), 0.58 (6H, q, J = 8.0 Hz); ¹³C NMR (125 MHz, CDCl₃) δ 212.11 (0), 73.46 (0), 61.90 (1), 53.21 (1), 50.04 (0), 43.50 (2), 41.00 (2), 40.66 (1), 38.44 (2), 35.50 (1), 30.09 (3), 29.62 (3), 27.17 (2), 24.81 (2), 24.03 (2), 19.01 (2), 18.29 (3), 13.19 (3), 12.49 (3), 7.14 (3), 6.81 (2); MS (EI) *m*/*z* no M⁺, 393 (9, M⁺ - CH₃), 379 (34, M⁺ - Et), 350 (17), 335 (2), 293 (2), 259 (34), 239 (6), 225 (3), 206 (7), 191 (38), 173 (100), 149 (16), 135 (80), 103 (80), 75 (67); MS (ESI) *m*/*z* 431 (34, [M+Na]⁺), 839 (100, [2M+Na]⁺), 1248 (28, [3M+H+Na]⁺); exact mass calculated for C₂₅H₄₈O₂SiNa [M+Na]⁺ 431.3321, found 431.3316.

### (20S,22R)-2-Methylene-19-nor-22-methyl-1α,25-dihydroxyvitamin D₃ (IIA)

Phenyllithium (1.8 M in di-n-butyl ether, 45 µL, 6.8 mg, 0.081 mmol) was added to a stirred solution of the phosphine oxide **20** (50 mg, 86 µmol) in anhydrous THF (400 µL) at - 30 °C. After 30 min the mixture was cooled to -78 °C and a precooled solution of the ketone **19A** (14 mg, 34 µmol) in anhydrous THF (300 + 200 µL) was added. The reaction mixture was stirred under argon at -78 °C for 4 hours and then at +4 °C for 19 h. Ethyl acetate was added and the organic phase was washed with brine, dried (Na₂SO₄) and concentrated. The residue was applied to a Waters silica Sep-Pak cartridge (5 g). The cartridge was washed with hexane and ethyl acetate/hexane (1:99) to give the protected vitamin **21A** (23.89 mg, 90% yield).
UV (in hexane) λmax 263.0, 253.0, 245.0 nm; ¹H NMR (500 MHz, CDCl₃) δ 6.22 (1H, d, J = 11.1 Hz, 6-H), 5.84 (1H, d, J = 11.1 Hz, 7-H), 4.97 (1H, s, =CH₂), 4.92 (1H, s, =CH₂), 4.43 (2H, m, 1β-H and 3α-H), 2.83 (1H, dm, J = 12.4 Hz), 2.52 (1H, dd, J = 13.3, 5.8 Hz, 10α-H), 2.46 (1H, dd, J = 12.5, 4.3 Hz, 4α-H), 2.33 (1H, dd, J = 13.3, 2.9 Hz, 10β-H), 2.18 (1H, dd, J = 12.5, 8.3 Hz, 4β-H), 2.00 (2H, m), 1.187 and 1.180 (each 3H, each s, 26-H₃, 27-H₃), 0.94 (9H, t, J = 7.9 Hz), 0.896 (9H, s, t-BuSi), 0.865 (9H, s, t-BuSi), 0.762 (3H, d, J = 6.7 Hz, 28-H₃), 0.706 (3H, d, J = 5.8 Hz, 21-H₃), 0.561 (6H, q, J = 7.9 Hz), 0.535 (3H, s, 18-H₃), 0.080 (3H, s, SiMe), 0.067 (3H, s, SiMe), 0.049 (3H, s, SiMe), 0.026 (3H, s, SiMe); ¹³C NMR (125 MHz, CDCl₃) δ 152.98 (0, C-2), 141.24 (0, C-8), 132.72 (0, C-5), 122.42 (1, C-6), 116.13 (1, C-7), 106.25 (2, =CH₂), 73.50 (0, C-25), 72.53 and 71.63 (each 1, C-1, C-3), 56.35 (1), 53.54 (1), 47.61 (2), 45.73 (0, C-13), 43.33 (2), 40.28 (2), 39.03 (1), 38.56 (2), 35.03 (1), 30.37 (2), 29.89 and 29.85 (each 3, C-26, C-27), 28.78 (2), 27.88 (2), 25.84 (3), 25.77 (3), 23.44 (2), 22.10 (2), 18.25 (0), 18.16 (0), 13.93 (3), 12.24 (3), 11.96 (3), 7.13 (3), 6.82 (2), -4.87 (3), -5.10 (3); MS (ESI) m/z 795 (20, [M+Na⁺]); exact mass (ESI) calculated for C₄₆H₈₈O₃Si₃Na [M+Na]⁺ 795.5939, found 795.5946.

The protected vitamin **21A** (23.89 mg, 30.9 µmol) was dissolved in THF (4 mL) and acetonitrile (3 mL). A solution of aqueous 48% HF in acetonitrile (1:9 ratio, 4 mL) was added at 0 °C and the resulting mixture was stirred at room temperature for 2 h. Saturated aqueous NaHCO₃ solution was added and the reaction mixture was extracted with dichloromethane. The combined organic phases were dried (Na₂SO₄) and concentrated under reduced pressure. The residue was diluted with 2 mL of hexane/ethyl acetate (7:3) and applied to a Waters silica Sep-Pak cartridge (5 g). An elution with hexane/ethyl acetate (7:3, then 1:1) gave the crude product **IIA**. The vitamin **IIA** was further purified by straight phase HPLC [9.4 x 250 mm Zorbax Silica column, 4 mL/min, hexane/2-propanol (85:15) solvent system, Rₜ = 7.9 min.] and reverse phase HPLC [9.4 x 250 mm Zorbax RX-C18 column, 3 mL/min, methanol/water (85:15) solvent system, Rₜ = 14.7 min.] to give the pure compound **IIA** (10.285 mg, 77% yield). m.p. 117 °C (Et₂O); UV (in EtOH) λₘₐₓ 261.5, 252.0, 244.5 nm; ¹H NMR (500 MHz, CDCl₃) δ 6.35 (1H, d, J = 11.2 Hz, 6-H), 5.89 (1H, d, J = 11.2 Hz, 7-H), 5.11 (1H, s, =CH₂), 5.08 (1H, s, =CH₂), 4.46 (2H, m, 1β-H and 3α-H), 2.85 (1H, dd, J = 13.8, 4.4 Hz, 4α-H), 2.82 (1H, m), 2.56 (1H, dd, J = 13.3, 3.5 Hz, 10β-H), 2.33 (1H, dd, J = 13.3, 6.0 Hz, 10α-H), 2.29 (1H, dd, J = 13.8, 8.4 Hz, 4β-H), 1.21 (6H, s, 26-H₃, 27-H₃), 0.78 (3H, d, J = 6.7 Hz, 28-H₃), 0.71 (3H, d, J = 5.7 Hz, 21-H₃), 0.54 (3H, s, 18-H₃); ¹³C NMR (125 MHz, CDCl₃) δ 151.98 (0, C-2), 143.25 (0, C-8), 130.52 (0, C-5), 124.14 (1, C-6), 115.36 (1, C-7), 107.69 (2, =CH₂), 71.76 (1), 71.14 (0), 70.58 (1), 56.34 (1), 53.48 (1), 45.80 (0), 45.74 (2), 42.11 (2), 40.08 (2), 38.81 (1), 38.12 (2), 34.96 (1), 30.24 (2), 29.26 (3), 29.12 (3), 28.93 (2), 27.78 (2), 23.44 (2), 22.11 (2), 13.88 (3), 12.14 (3), 12.04 (3); MS (EI) m/z no M⁺, 401 (100, M⁺ - Et), 383 (52, M⁺ - Et - H₂O), 351 (15), 314 (14), 289 (39), 272 (27), 236 (38), 202 (10), 173 (19), 144 (42), 120 (95), 94 (59); MS (ESI) *m*/*z* 453 (100, [M+Na]⁺), 883 (25, [2M+Na]⁺), 1314 (5, [3M+H+Na]⁺); exact mass calculated for C₂₈H₄₆O₃Na [M+Na]⁺ 453.3345 found 453.3329.

### (20S,22S)-2-Methylene-19-nor-22-methyl-1α,25-dihydroxyvitamin D₃ (IIB)

Phenyllithium (1.83 M in di-n-butyl ether, 50 µL, 7.7 mg, 0.091 mmol) was added to a stirred solution of the phosphine oxide **20** (55 mg, 86 µmol) in anhydrous THF (400 µL) at - 30 °C. After 30 min the mixture was cooled to -78 °C and a precooled solution of the ketone **19B** (14.4 mg, 35 µmol) in anhydrous THF (300 + 200 µL) was added. The reaction mixture was stirred under argon at -78 °C for 4 hours and then at +4 °C for 19 h. Ethyl acetate was added and the organic phase was washed with brine, dried (Na₂SO₄) and concentrated. The residue was applied to a Waters silica Sep-Pak cartridge (5 g). The cartridge was washed with hexane and ethyl acetate/hexane (2:98) to give the protected vitamin **21B** (23.618 mg, 87% yield).
UV (in hexane) λmax 263.0, 253.5, 245.5 nm; ¹H NMR (500 MHz, CDCl₃) δ 6.22 (1H, d, J = 11.2 Hz, 6-H), 5.84 (1H, d, J = 11.2 Hz, 7-H), 4.97 (1H, s, =CH₂), 4.92 (1H, s, =CH₂), 4.43 (2H, m, 1β-H and 3α-H), 2.83 (1H, dm, J = 12.5 Hz), 2.52 (1H, dd, J = 13.2, 6.1 Hz, 10α-H), 2.46 (1H, dd, J = 12.7, 4.1 Hz, 4α-H), 2.33 (1H, dd, J = 13.2, 2.9 Hz, 10β-H), 2.18 (1H, dd, J = 12.7, 8.4 Hz, 4β-H), 2.00 (1H, m), 1.19 (6H, s, 26-H₃, 27-H₃), 0.95 (9H, t, J = 7.9 Hz), 0.897 (9H, s, t-BuSi), 0.865 (9H, s, t-BuSi), 0.84 (3H, d, J = 6.8 Hz), 0.75 (3H, d, J = 6.8 Hz), 0.57 (6H, q, J = 7.9 Hz), 0.53 (3H, s, 18-H₃), 0.080 (3H, s, SiMe), 0.067 (3H, s, SiMe), 0.049 (3H, s, SiMe), 0.026 (3H, s, SiMe); ¹³C NMR (125 MHz, CDCl₃) δ 152.98 (0, C-2), 141.24 (0, C-8), 132.71 (0, C-5), 122.43 (1, C-6), 116.08 (1, C-7), 106.25 (2, =CH₂), 73.57 (0, C-25), 72.53 and 71.63 (each 1, C-1, C-3), 56.21 (1), 53.17 (1), 47.61 (2), 45.74 (0, C-13), 43.50 (2), 41.31 (1), 40.09 (2), 38.55 (2), 35.34 (1), 29.96 (3) and 29.73 (each 3, C-26 and C-27), 28.80 (2), 27.45 (2), 25.84 (3), 25.78 (3), 24.82 (2), 23.44 (2), 22.17 (2), 18.43 (3), 18.25 (0), 18.16 (0), 13.17 (3), 12.10 (3), 7.15 (3), 6.82 (2), -4.87 (3), -5.10 (3).

The protected vitamin **21B** (23.518 mg, 30.5 µmol) was dissolved in THF (4 mL) and acetonitrile (3 mL). A solution of aqueous 48% HF in acetonitrile (1:9 ratio, 4 mL) was added at 0 °C and the resulting mixture was stirred at room temperature for 2 h. A saturated aqueous NaHCO₃ solution was added and the reaction mixture was extracted with dichloromethane. The combined organic phases were dried (Na₂SO₄) and concentrated under reduced pressure. The residue was diluted with 2 mL of hexane/ethyl acetate (7:3) and applied to a Waters silica Sep-Pak cartridge (5 g). An elution with hexane/ethyl acetate (7:3, then 1:1) gave the crude product **IIB**. The vitamin **IIB** was further purified by straight phase HPLC [9.4 x 250 mm Zorbax Silica column, 4 mL/min, hexane/2-propanol (85:15) solvent system, Rt = 7.3 min.] and reverse phase HPLC [9.4 x 250 mm Zorbax RX-C18 column, 3 mL/min, methanol/water (85:15) solvent system, Rt = 11.7 min.] to give the vitamin **IIB** (6.56 mg, 50% yield) and its (22R) epimer (2.92 mg, 22% yield). UV (in EtOH) λₘₐₓ 261.5, 252.5, 245.0 nm; ¹H NMR (500 MHz, CDCl₃) δ 6.35 (1H, d, J = 11.2 Hz, 6-H), 5.89 (1H, d, J = 11.2 Hz, 7-H), 5.11 (1H, s, =CH₂), 5.09 (1H, s, =CH₂), 4.46 (2H, m, 1β-H and 3α-H), 2.85 (1H, dd, J = 13.0, 4.4 Hz, 4α-H), 2.82 (1H, dm, J = 13.7 Hz), 2.57 (1H, dd, J = 13.4, 3.8 Hz, 10β-H), 2.33 (1H, dd, J = 13.4, 6.2 Hz, 10α-H), 2.29 (1H, dd, J = 13.0, 8.4 Hz, 4β-H), 2.03 (1H, m), 1.91 (dm, J = 12.1 Hz), 1.22 (6H, s, 26-H₃, 27-H₃), 0.86 (3H, d, J = 6.8 Hz), 0.76 (3H, d, J = 6.8 Hz), 0.54 (3H, s, 18-H₃); ¹³C NMR (125 MHz, CDCl₃) δ 151.96 (0, C-2), 143.31 (0, C-8), 130.46 (0, C-5), 124.22 (1, C-6), 115.32 (1, C-7), 107.71 (2, =CH₂), 71.79 and 70.66 (each 1, C-1, C-3), 71.25 (0, C-25), 56.21 (1), 53.06 (1), 45.86 (0, C-13), 45.78 (2), 42.36 (2), 41.15 (1), 39.93 (2), 38.14 (2), 35.40 (1), 29.19 (3, C-26 and C-27), 28.95 (2), 27.37 (2), 24.80 (2), 23.47 (2), 22.23 (2), 18.32 (3), 13.20 (3), 12.14 (3); MS (EI) *m*/*z* 430 (9, M⁺), 412 (3, M⁺ - H₂O), 328 (7), 313 (8), 297 (5), 251 (5), 227 (3), 211 (5), 194 (48), 161 (12), 135 (51), 105 (100); exact mass calculated for C₂₈H₄₆O₃ [M]⁺ 430.3447 found 430.3447.

### Comparative Example 1B: Synthesis of (20R,22S)-2-methylene-19-nor-22-methyl-1α,25-dihydroxyvitamin D₃ and (20R,22R)-2-methylene-19-nor-22-methyl-1α,25-dihydroxyvitamin D₃

Comparative compounds of formula I, formula II, formula IIC and formula IID were prepared using the methods shown in Schemes 4 and 5. As shown in Scheme 4, Compound **4** was reacted with sodium cyanide in DMSO to give cyanide **22.** The cyano compound **22** was then treated with 4-bromo-2-methyl-1-triethylsilyloxy butane **(11),** in presence of a mixture of n-butyllithium and diisopropylamine, to provide compound **23.** The cyano group of compound **23** was converted to the corresponding aldehyde **24** by treating it with diisobutylaluminum hydride in dichloromethane. Aldehyde **24** was then reduced to alcohol **25** using sodium borohydride in methanol. The free hydroxyl group of compound **25** was then reacted with tosyl chloride in pyridine and the resulting tosyl protected compound **26** was reduced to the corresponding alkane **27** using lithium aluminum hydride as the reducing agent. The triethylsilyl protected dihydroxy compound **27** was then deprotected using tetrabutylammonium fluoride in THF and the racemic mixture of diols thus obtained was separated by crystallization from ethyl acetate to provide the two separate isomers, the 22S **28** diol and 22R diol **29**. Each of the diols **28** and **29** were then separately oxidized (TPAP/4-MMO or PDC/PPTS) to produce the respective ketones. Each ketone was further independently treated with triethylsilyl trifluoromethanesulfonate and 2,6-lutidine in dichloromethane to provide the triethylsilyl protected ketone 22S compound **30A** or 22R compound **30B.** 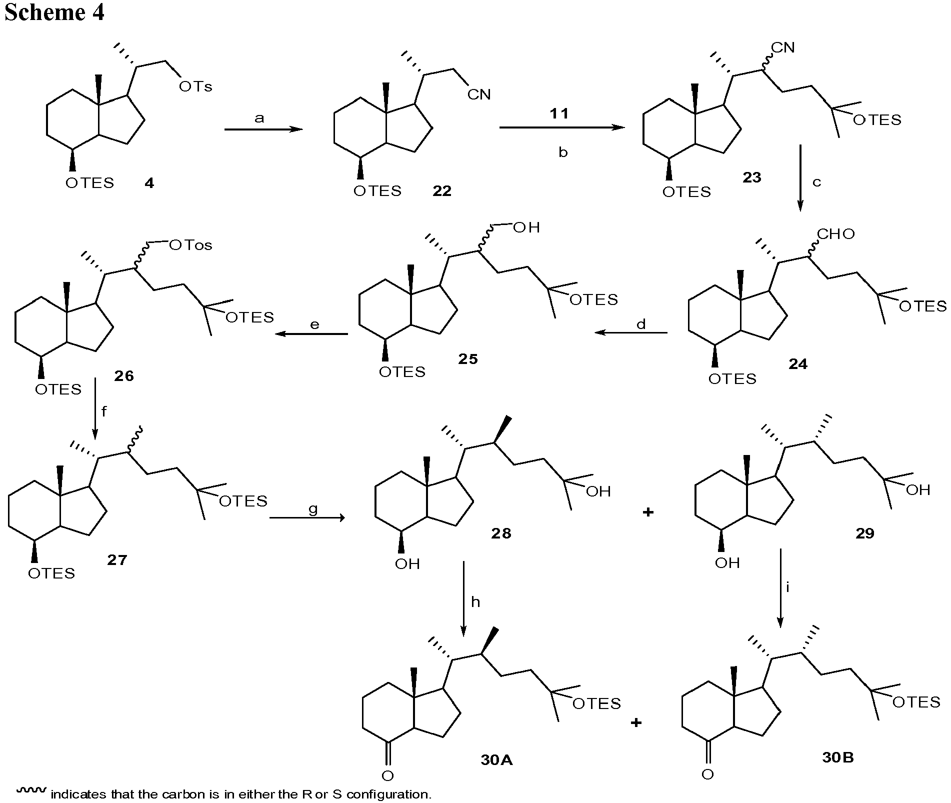
a) NaCN, DMSO (**22**, 97%)
b) 1. n-BuLi, DIPA, THF; 2. **11** (**23**, 93%)
c) DIBAL, toluene, DCM (**24**, 79%)
d) NaBH₄, MeOH (**25**, 71%)
e) TosCl, pyridine (**26**, 92%)
f) LiAlH₄, DEE (**27**, 80%)
g) 1. TBAF, THF (**28** and **29**, 99%); 2. Crystallization from EtOAc
h) 1. PDC, PPTS, DCM; 2. TESOTf, 2,6-lutidine, DCM (**30A**, 53%)
i) 1. Mol sieves 4Å, 4-MMO, TPAP, DCM; 2. TESOTf, 2,6-lutidine, DCM (**30B**, 95%)

Scheme 5 illustrates the conversion of compounds **30A** or **30B** to compounds **IIC** or **IID.** A Wittig-Horner condensation of the protected Grundmann's Ketone (compound **30A** or **30B)** with the phosphine oxide (compound **20**) in the presence of phenyllithium was performed as shown is Scheme 5. Finally, the target compound (compound **IIC** or **IID**) was generated by deprotection of hydroxy groups in compounds **31A** or **31 B** in the presence of hydrofluoric acid.

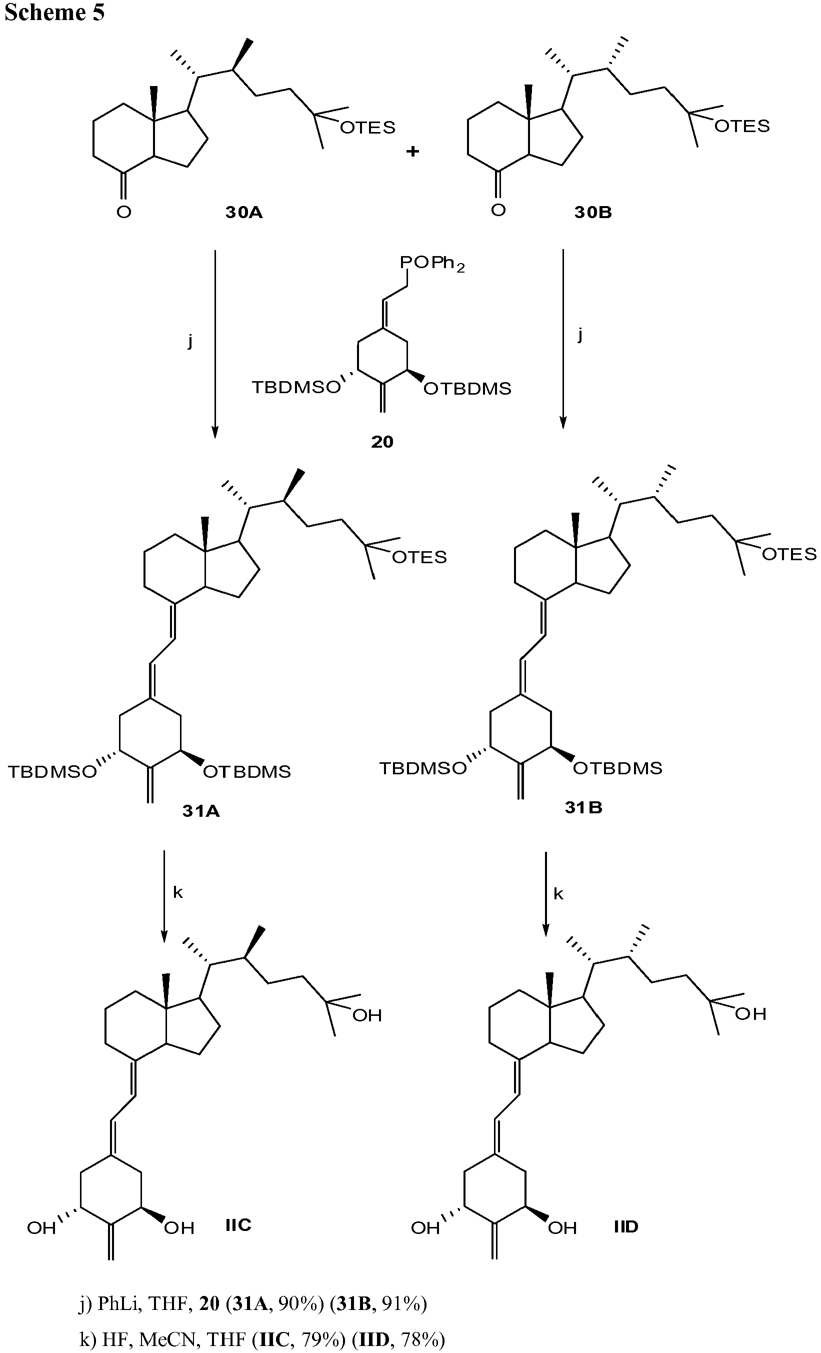

### (8S,20S)-Des-A,B-8-[(triethylsilyl)oxy]-20-(cyanomethyl)-pregnane (22)

Sodium cyanide (2 g, 41 mmol) was added to a solution of tosylate **4** (0.84 g, 1.75 mmol) in dry DMSO (8 mL). The resulting mixture was stirred at 90 °C for 3 h, then it was cooled, diluted with water and extracted with ethyl acetate. Combined organic phases were dried (Na₂SO₄) and concentrated. The residue was purified by column chromatography on silica gel (10% ethyl acetate/hexane) to give the cyanide **22** (0.57 g, 97% yield) as a colorless oil.
[α]_{D} +16.6° (c 1.0, CHCl₃); ¹H NMR (400 MHz, CDCl₃) δ 4.04 (1H, d, J = 2.1 Hz), 2.34 (1H, dd, J = 16.6, 3.7 Hz), 2.23 (1H, dd, J = 16.6, 7.0 Hz), 1.92 (1H, m), 1.13 (3H, d, J = 6.6 Hz), 0.942 (9H, t, J = 7.9 Hz), 0.921 (3H, s), 0.55 (6H, q, J = 7.9 Hz); ¹³C NMR (125 MHz, CDCl₃) δ 119.09 (0), 69.12 (1), 55.34 (1), 52.86 (1), 42.18 (0), 40.35 (2), 34.40 (2), 33.09 (1), 27.19 (2), 24.69 (2), 22.82 (2), 19.23 (3), 17.53 (2), 13.63 (3), 6.91 (3), 4.89 (2); MS (EI) *m*/*z* 335 (10), 320 (3), 306 (100), 292 (28), 225 (7), 202 (20), 188 (10), 161 (17), 135 (14), 103 (55); exact mass calculated for C₂₀H₃₇ONSi (M⁺) 335.2644, found 335.2656.

### (8S,20R,22ξ)-Des-A,B-8-[(triethylsilyl)oxy]-22-cyano-25-[(triethylsilyl)oxy]-cholestane (23)

n-Butyllithium (1.6 M in hexane, 2.7 mL, 0.28 g, 4.32 mmol) was added to a solution of diisopropylamine (0.6 mL, 0.43 g, 4.25 mmol) in THF (4 mL) at 0 °C. The resulting mixture was stirred at 0 °C for 30 min, then it was cooled to -78 °C and a solution of cyanide **22** (0.57 g, 1.70 mmol) in THF (5 mL) was added. The mixture was stirred at -78 °C for 30 min and a solution of bromide **11** (0.96 g, 3.42 mmol) was added. The reaction mixture was stirred at -78 °C for 1 h and then at 0 °C for 1 h. It was quenched with a saturated aqueous NH₄Cl solution and extracted with ethyl acetate. Combined organic phases were washed with brine and dried (Na₂SO₄) and concentrated. The residue was purified by column chromatography on silica gel (1.5%, 3% and 10% ethyl acetate/hexane) to give a mixture of cyanides **23** (0.85 g, 93% yield).
Major isomer: ¹H NMR (400 MHz, CDCl₃) δ 4.03 (1H, s), 2.56 (1H, m), 1.22 (3H, s), 1.21 (3H, s), 1.04 (3H, d, J = 6.6 Hz), 0.944 (18H, t, J = 7.8 Hz), 0.923 (3H, s), 0.57 (6H, q, J = 7.8 Hz), 0.55 (6H, q, J = 7.8 Hz); Minor isomer (visible signals): ¹H NMR (400 MHz, CDCl₃) δ 1.08 (3H, d, J = 6.8 Hz); MS (EI) *m*/*z* 492 (36), 478 (6), 390 (11), 374 (96), 351 (53), 322 (11), 271 (18), 225 (13), 201 (23), 185 (25), 173 (75), 131 (51), 103 (100); MS (ESI) *m*/*z* 558 (30, [M+Na]⁺), 1093 (20, [2M+Na]⁺); exact mass calculated for C₃₁H₆₁NO₂Si₂Na [M+Na]⁺ 558.4139, found 558.4141.

### (8S,20R,22ξ)-Des-A,B-8-[(triethylsilyl)oxy]-22-formyl-25-[(triethylsilyl)oxy]-cholestane (24)

Diisobutylaluminum hydride (1.5 M in toluene, 1.4 mL, 0.3 g, 2.1 mmol) was added to a solution of cyanides **23** (0.81 g, 1.51 mmol) in dichloromethane (10 mL) at -10 °C. The reaction mixture was stirred at -10 °C for 1 hour, then it was quenched with a saturated aqueous sodium potassium tartrate solution (5 mL). The water phase was extracted with dichloromethane. Combined organic layers were washed with brine and dried (Na₂SO₄) and concentrated. The residue was purified by column chromatography on silica gel (3% ethyl acetate/hexane) to give a mixture of aldehydes **24** (0.64 g, 79% yield).
Major isomer: ¹H NMR (400 MHz, CDCl₃) δ 9.72 (1H, d, J = 3.2 Hz), 4.03 (1H, br s), 1.20 (6H, s), 1.02 (3H, d, J = 7.0 Hz), 0.944 (9H, t, J = 7.8 Hz), 0.939 (9H, t, J = 7.8 Hz), 0.920 (3H, s), 0.563 (6H, q, J = 7.8 Hz), 0.554 (6H, q, J = 7.8 Hz); Minor isomer (visible signals): ¹H NMR (400 MHz, CDCl₃) δ 9.63 (1H, s); MS (EI) *m*/*z* 453 (1), 377 (5), 353 (8), 321 (18), 295 (8), 257 (20), 201 (53), 173 (88), 163 (43), 135 (26), 115 (59), 103 (100); MS (ESI) *m*/*z* 561 (80, [M+Na]⁺), 1099 (40, [2M+Na]⁺); exact mass calculated for C₃₁H₆₂O₃Si₂Na [M+Na]⁺ 561.4135 found 561.4139.

### (8S,20R,22ξ)-Des-A,B-8-[(triethylsilyl)oxy]-22-(hydroxymethyl)-25-[(triethylsilyl)oxy]-cholestane (25)

Sodium borohydride (0.44 g, 11.63 mmol) was added to a solution of aldehydes **24** (0.64 g, 1.19 mmol) in methanol (10 mL) at 0 °C. The reaction mixture was warmed to room temperature and stirred for 2 h, then it was quenched with water and extracted with ethyl acetate. Combined organic layers were washed with brine and dried (Na₂SO₄) and concentrated. The residue was purified by column chromatography on silica gel (3%, 10% ethyl acetate/hexane) to give a mixture of alcohols **25** (0.46 g, 71% yield) as a colorless oil.
Major isomer: ¹H NMR (500 MHz, CDCl₃) δ 4.03 (1H, br s), 3.71 (1H, dd, J = 10.7, 4.2 Hz), 3.39 (1H, dd, J = 10.7, 8.0 Hz), 1.205 (6H, s), 0.946 (18H, t, J = 7.9 Hz), 0.909 (3H, s), 0.798 (3H, d, J = 7.1 Hz), 0.568 (6H, q, J = 7.9 Hz), 0.551 (6H, q, J = 7.9 Hz); Minor isomer (visible signals): ¹H NMR (500 MHz, CDCl₃) δ 3.61 (1H, dd, J = 10.8, 4.8 Hz), 3.46 (1H, dd, J = 10.8, 9.2 Hz), 0.784 (1H, d, J = 7.3 Hz); MS (EI) *m*/*z* 453 (1), 425 (2), 391 (40), 340 (5), 311 (57), 297 (27), 259 (35), 225 (37), 207 (24), 191 (40), 173 (72), 163 (46), 135 (100); MS (ESI) *m*/*z* 563 (100, [M+Na]⁺), 1103 (50, [2M+Na]⁺); exact mass calculated for C₃₁H₆₄O₃Si₂Na [M+Na]⁺ 563.4292 found 563.4298.

### (8S,20R,22ξ)-Des-A,B-8-[(triethylsilyl)oxy]-22-methyl-25-[(triethylsilyl)oxy]-cholestane (27)

A solution of tosyl chloride (0.66 g, 3.46 mmol) in pyridine (2 mL) was added to a mixture of alcohols **25** (0.46 g, 0.85 mmol) in dry pyridine (4 mL) at -20 °C. The reaction mixture was stirred at -20 °C for 1 hour and at +4 °C for 18 h. Then it was pulled into a saturated aqueous CuSO₄ solution and extracted with dichloromethane. Combined organic phases were dried (Na₂SO₄) and concentrated. The residue was purified by column chromatography on silica gel (3% ethyl acetate/hexane) to give a mixture of tosylates **26** (0.54 g, 92% yield). LiAlH₄ (0.4 g, 10.53 mmol) was added to a solution of tosylates **26** (0.53 g, 0.76 mmol) in dry diethyl ether (10 mL) at 0 °C. The reaction mixture was stirred at +4 °C for 20 h. The excess of LiAlH₄ was decomposed with water. The reaction mixture was diluted with diethyl ether and then it was filtered through Celite. The filtrate was extracted with ethyl acetate, dried (Na₂SO₄) and concentrated. The residue was purified by column chromatography on silica gel (3% ethyl acetate/hexane) to give a mixture of products **27** (0.32 g, 80% yield).
Major isomer: ¹H NMR (400 MHz, CDCl₃) δ 4.03 (1H, br s), 1.94 (1H, m), 1.182 (6H, s), 0.952 (18H, t, J = 7.9 Hz), 0.917 (3H, s), 0.733 (3H, d, J = 6.6 Hz), 0.690 (3H, d, J = 6.7 Hz), 0.565 (6H, q, J = 7.9 Hz), 0.556 (6H, q, J = 7.9 Hz); Minor isomer (visible signals): ¹H NMR (400 MHz, CDCl₃) δ 0.902 (3H, s), 0.843 (3H, d, J = 6.8 Hz), 0.764 (3H, d, J = 6.5 Hz); MS (EI) *m*/*z* 496 (62), 481 (6), 391 (11), 363 (60), 259 (28), 246 (42), 225 (25), 173 (90), 135 (66), 103 (100); MS (ESI) *m*/*z* 547 (5, [M+Na]⁺); exact mass calculated for C₃₁H₆₄O₂Si₂Na [M+Na]⁺ 547.4343 found 547.4355.

### (8S,20R,22S)-Des-A,B-22-methyl-cholestan-8,25-diol (28) and (8S,20R,22R)-Des-A,B-22-methyl-cholestan-8,25-diol (29)

Tetrabutylammonium fluoride (1.0 M in THF, 3.4 mL, 3.4 mmol) was added to a solution of compounds **27** (0.31 g, 0.59 mmol) in THF (3 mL) at 0 °C. The reaction mixture was stirred at +4 °C for 20 h, then it was diluted with water and extracted with ethyl acetate. Combined organic extracts were dried (Na₂SO₄) and concentrated. The residue was purified by column chromatography on silica gel (10%, 50% ethyl acetate/hexane) to give a mixture of diols **28** and **29** (0.17 g, 99% yield) in 2:1 ratio, respectively (based on ¹H NMR). Isomers were separated by crystallization from ethyl acetate and absolute configuration was established by X-ray analysis. Pure crystals (96 mg) of the isomer **28** were obtained after first crystallization and the 22S absolute configuration of the diol **28** was established. Pure crystals (44.6 mg) of the other isomer 29 were obtained from the filtrate after first crystallization and the 22R absolute configuration of the diol **29** was established. A second batch of pure crystals (16 mg) of the diol **28** was obtained from the filtrate after second crystallization.
**28:** [α]_{D} +15.4° (c 1.0, CHCl₃); m.p. 147-148 °C (EtOAc); ¹H NMR (500 MHz, CDCl₃) δ 4.07 (1H, s), 1.98 (1H, dm, J = 12.8 Hz), 1.209 (6H, s), 0.934 (3H, s), 0.750 (3H, d, J = 6.7 Hz), 0.711 (3H, d, J = 6.8 Hz); ¹³C NMR (100 MHz, CDCl₃) δ 71.13 (0), 69.42 (1), 54.26 (1), 52.63 (1), 42.18 (2), 41.78 (0), 40.50 (2), 38.14 (1), 34.84 (1), 33.59 (2), 30.26 (2), 29.28 (3), 29.19 (3), 26.72 (2), 22.42 (2), 17.45 (2), 13.47 (3), 13.08 (3), 12.19 (3); MS (EI) *m*/*z* no M⁺, 277 (45), 259 (36), 244 (23), 216 (16), 189 (19), 178 (35), 162 (72), 151 (33), 134 (100), 135 (33), 111 (72); MS (ESI) *m*/*z* 319 (60, [M+Na]⁺), 615 (100, [2M+Na]⁺), 911 (15, [3M+Na]⁺); exact mass calculated for C₁₉H₃₆O₂Na (MNa⁺) 319.2613, found 319.2621.
**29:** [α]_{D} +34.0° (c 1.0, CHCl₃); m.p. 108-110 °C (EtOAc); ¹H NMR (500 MHz, CDCl₃) δ 4.06 (1H, s), 1.97 (1H, dm, J = 12.9 Hz), 1.209 (3H, s), 1.199 (3H, s), 0.922 (3H, s), 0.866 (3H, d, J = 6.8 Hz), 0.779 (3H, d, J = 6.6 Hz); ¹³C NMR (125 MHz, CDCl₃) δ 71.17 (0), 69.39 (1), 54.25 (1), 52.57 (1), 42.78 (2), 41.78 (0), 40.89 (1), 40.46 (2), 35.03 (1), 33.60 (2), 29.55 (3), 29.00(3), 26.82 (2), 23.70 (2), 22.45 (2), 18.89 (3), 17.45 (2), 13.45 (3), 12.87 (3); MS (EI) *m*/*z* no M⁺, 278 (53), 260 (22), 245 (17), 217 (7), 191 (12), 179 (13), 163 (52), 151 (31), 135 (48), 111 (100); MS (ESI) *m*/*z* 319 (45, [M+Na]⁺), 615 (55, [2M+Na]⁺), 911 (10, [3M+Na]⁺); exact mass calculated for C₁₉H₃₆O₂Na (MNa⁺) 319.2613, found 319.2619.

### (20R,22S)-Des-A,B-22-methyl-25-[(triethylsilyl)oxyl-cholestan-8-one (30A)

Pyridinium dichromate (0.18 g, 0.48 mmol) and pyridinium p-toluenesulfonate (24 mg, 95 µmol) were added in one portion to a solution of diol **28** (24.9 mg, 84 µmol) in dry dichloromethane (5 mL). The reaction mixture was stirred at room temperature for 1 hour 15 min, then it was quenched with water and extracted with dichloromethane. Combined organic layers were dried (Na₂SO₄) and concentrated. The residue was applied to a Waters silica Sep-Pak cartridge (2g). Elution with dichloromethane gave the ketone (23.6 mg). Triethylsilyl trifluoromethanesulfonate (25 µL, 29.2 mg, 0.11 mmol) was added dropwise to a solution of the ketone (23.6 mg) and 2,6-lutidine (30 µL, 27.6 mg, 0.26 mmol) in dry dichloromethane (2 mL) at -40 °C. The reaction mixture was stirred at -40 °C for 15 min, then it was diluted with dichloromethane and washed with water. The organic layer was dried (Na₂SO₄) and concentrated. The residue was applied to a Waters silica Sep-Pak cartridge (10g). Elution with ethyl acetate/hexane (2:98, then 5:95) gave the protected ketone **30A** (18.2 mg, 53% yield in 2 steps).
[α]_{D} -7.8° (*c* 1.0, CHCl₃); ¹H NMR (400 MHz, CDCl₃) δ 2.46 (1H, dd, J = 11.4, 7.5 Hz), 1.176 (6H, s), 0.935 (9H, t, J = 7.9 Hz), 0.797 (3H, d, J = 6.6 Hz), 0.719 (3H, d, J = 6.7 Hz), 0.643 (3H, s), 0.553 (6H, q, J = 7.9 Hz); ¹³C NMR (100 MHz, CDCl₃) δ 212.20 (0), 73.38 (0), 62.01 (1), 54.47 (1), 49.90 (0), 43.25 (2), 40.98 (2), 39.09 (2), 38.43 (1), 35.00 (1), 30.19 (2), 29.86 (3), 29.82 (3), 27.17 (2), 24.09 (2), 18.96 (2), 13.14 (3), 12.44 (3), 12.37 (3), 7.10 (3), 6.77 (2); MS (EI) *m*/*z no* M⁺, 393 (13), 379 (38), 350 (35), 259 (43), 203 (17), 173 (100), 163 (64), 135 (84), 103 (99); MS (ESI) *m*/*z* 431 (2, [M+Na]⁺), 839 (20, [2M+Na]⁺), 1248 (60, [3M+H+Na]⁺); exact mass calculated for C₂₅H₄₈O₂SiNa (MNa⁺) 431.3321, found 431.3318.

### (20R,22R)-Des-A,B-22-methyl-25-[(triethylsilyl)oxyl-cholestan-8-one (30B)

Molecular sieves 4Å (60 mg) were added to a solution of 4-methylmorpholine oxide (33 mg, 0.282 mmol) in dichloromethane (0.25 mL). The mixture was stirred at room temperature for 15 min and tetrapropylammonium perruthenate (2 mg, 5.7 µmol) was added, followed by a solution of diol **29** (16 mg, 54 µmol) in dichloromethane (300 + 250 µL). The resulting suspension was stirred at room temperature for 1 h. The reaction mixture was filtered through a Waters silica Sep-Pak cartridge (2 g) that was further washed with ethyl acetate. After removal of the solvent, the ketone (14.4 mg, 89% yield) was obtained as a colorless oil.
Triethylsilyl trifluoromethanesulfonate (20 µL, 23 mg, 88 µmol) was added dropwise to a solution of the ketone (14.4 mg, 49 µmol) and 2,6-lutidine (20 µL, 18 mg, 0.17 mmol) in dichloromethane (2 mL) at -40 °C. The reaction mixture was stirred at -40 °C for 15 min, then it was diluted with dichloromethane and washed with water. The organic layer was dried (Na₂SO₄) and concentrated. The residue was applied to a Waters silica Sep-Pak cartridge (5 g). Elution with ethyl acetate/hexane (1:99, then 2:98) gave the protected ketone **30B** (19 mg, 95% yield). [α]_{D} +3.4 (c 1.0, CHCl₃); ¹H NMR (400 MHz, CDCl₃) δ 2.45 (1H, dd, J = 11.4, 7.6 Hz), 1.207 (3H, s), 1.183 (3H, s), 0.955 (9H, t, J = 7.9 Hz), 0.865 (3H, d, J = 6.8 Hz), 0.835 (3H, d, J = 6.8 Hz), 0.636 (3H, s), 0.569 (6H, q, J = 7.9 Hz); ¹³C NMR (100 MHz, CDCl₃) δ 212.19 (0), 73.49 (0), 62.01 (1), 54.55 (1), 49.87 (0), 43.90 (2), 41.28 (1), 40.99 (2), 39.12 (2), 35.31 (1), 30.42 (3), 29.46 (3), 27.28 (2), 24.10 (2), 23.61 (2), 18.96 (3 and 2), 13.06 (3), 12.37 (3), 7.14 (3), 6.83 (2); MS (EI) *m*/*z* no M⁺, 393 (12), 379 (68), 350 (30), 259 (14), 203 (8), 173 (100), 163 (36), 135 (45), 103 (73); exact mass calculated for C₂₃H₄₃O₂Si [M - Et]⁺ 379.3032, found 379.3032.

### Comparative (20R,22S)-2-Methylene-19-nor-22-methyl-1α,25-dihydroxyvitamin D₃ (IIC)

Phenyllithium (1.8 M in di-n-butyl ether, 50 µL, 7.56 mg, 90 µmol) was added to a stirred solution of the phosphine oxide **20** (51 mg, 88 µmol) in anhydrous THF (500 µL) at -30 °C. After 30 min the mixture was cooled to -78 °C and a precooled solution of the ketone **30A** (17.9 mg, 44 µmol) in anhydrous THF (300 + 200µL) was added. The reaction mixture was stirred under argon at -78 °C for 4 hours and then at +4 °C for 19 h. Ethyl acetate was added and the organic phase was washed with brine, dried (Na₂SO₄) and concentrated. The residue was applied to a Waters silica Sep-Pak cartridge (5g). The cartridge was washed with hexane and ethyl acetate/hexane (1:99) to give the protected vitamin **31A** (30.66 mg, 90% yield).
UV (in hexane) λₘₐₓ 262.5, 253.0, 245.0 nm; ¹H NMR (500 MHz, CDCl₃) δ 6.22 (1H, d, J = 11.1 Hz, 6-H), 5.84 (1H, d, J = 11.1 Hz, 7-H), 4.97 (1H, s, =CH₂), 4.92 (1H, s, =CH₂), 4.43 (2H, m, 1β-H and 3α-H), 2.83 (1H, dm, J = 12.3 Hz), 2.53 (1H, dd, J = 13.3, 5.9 Hz, 10α-H), 2.47 (1H, dd, J= 13.0, 4.5 Hz, 4α-H), 2.33 (1H, dd, J = 13.3, 2.7 Hz, 10β-H), 2.18 (1H, dd, J = 13.0, 8.4 Hz, 4β-H), 1.188 (6H, s, 26-H₃, 27-H₃), 0.949 (9H, t, J = 7.9 Hz), 0.900 (9H, s, t-BuSi), 0.875 (3H, d, J = 7.6 Hz, 21-H₃), 0.868 (9H, s, t-BuSi), 0.722 (3H, d, J = 6.7 Hz), 0.567 (6H, q, J = 7.9 Hz), 0.559 (3H, s, 18-H₃), 0.083 (3H, s, SiMe), 0.069 (3H, s, SiMe), 0.052 (3H, s, SiMe), 0.029 (3H, s, SiMe); ¹³C NMR (100 MHz, CDCl₃) δ 152.98 (0, C-2), 141.33 (0, C-8), 132.69 (0, C-5), 122.43 (1, C-6), 116.05 (1, C-7), 106.24 (2, =CH₂), 73.52 (0, C-25), 72.55 and 71.60 (each 1, C-1, C-3); 56.32 (1), 54.23 (1), 47.61 (2), 45.65 (0, C-13), 43.35 (2), 40.74 (2), 39.07 (1), 38.53 (2), 35.01 (1), 30.37 (2), 29.90 and 29.80 (each 3, C-26, C-27), 28.80 (2), 27.33 (2), 25.84 (3), 25.77 (3), 23.49 (2), 22.13 (2), 18.26 (0), 18.16 (0), 13.19 and 12.53 and 11.96 (each 3, C-21, C-28, C-18), 7.13 (3), 6.81 (2), -4.87 (3), -5.10 (3); MS (ESI) m/z 795 (100, [M+Na⁺]); exact mass (ESI) calculated for C₄₆H₈₈O₃Si₃Na [M+Na]⁺ 795.5939 found 795.5910.

The protected vitamin **31A** (30.66 mg, 39.7 µmol) was dissolved in THF (4 mL) and acetonitrile (3 mL). A solution of aqueous 48% HF in acetonitrile (1:9 ratio, 4 mL) was added at 0 °C and the resulting mixture was stirred at room temperature for 3.5 h. Saturated aqueous NaHCO₃ solution was added and the reaction mixture was extracted with dichloromethane. The combined organic phases were dried (Na₂SO₄) and concentrated under reduced pressure. The residue was diluted with 2 mL of hexane/ethyl acetate (7:3) and applied to a Waters silica Sep-Pak cartridge (5 g). An elution with hexane/ethyl acetate (7:3, then 1:1) gave the crude product **IIC.** The vitamin **IIC** was further purified by straight phase HPLC [9.4 x 250 mm Zorbax Silica column, 4 mL/min, hexane/2-propanol (85:15) solvent system, Rₜ 8.5 min.] and reverse phase HPLC [9.4 x 250 mm Zorbax RX-C18 column, 3 mL/min, methanol/water (85:15) solvent system, Rₜ 15.2 min.] to give the pure comparative compound **IIC** (13.52 mg, 79% yield).
UV (in EtOH) λₘₐₓ 261.5, 252.0, 244.5 nm; ¹H NMR (500 MHz, CDCl₃) δ 6.35 (1H, d, J = 11.3 Hz, 6-H), 5.88 (1H, d, J = 11.3 Hz, 7-H), 5.10 (1H, s, =CH₂), 5.08 (1H, s, =CH₂), 4.46 (2H, m, 1β-H and 3α-H), 2.85 (1H, dd, J = 13.1, 4.5 Hz, 10β-H), 2.82 (1H, dm, J = 15.9 Hz, 9β-H), 2.57 (1H, dd, J = 13.4, 3.6 Hz, 4α-H), 2.33 (1H, dd, J = 13.4, 6.1 Hz, 4β-H), 2.28 (1H, dd, J = 13.1, 8.4Hz, 10α-H), 2.00 (2H, m), 1.210 (6H, s, 26-H₃, 27-H₃), 0.78 (3H, d, J = 5.8 Hz, 21-H₃), 0.73 (3H, d, J = 6.8 Hz, 28-H₃), 0.554 (3H, s, 18-H₃); ¹³C NMR (125 MHz, CDCl₃) δ 151.97 (0, C-2), 143.43 (0, C-8), 130.41 (0, C-5), 124.23 (1, C-6), 115.27 (1, C-7), 107.70 (2, =CH₂), 71.15 (0, C-25), 71.81 and 70.63 (each 1, C-1, C-3); 56.34 (1), 54.19 (1), 45.75 (0, C-13), 45.75 (2), 42.17 (2), 40.58 (2), 39.04 (1), 38.16 (2), 35.01 (1), 30.28 (2), 29.26 (3), 29.20 (3), 28.99 (2), 27.25 (2), 23.52 (2), 22.17 (2), 13.07 and 12.49 and 12.02 (each 3, C-21, C-28, C-18); MS (EI) m/z 430 (62, M⁺), 412 (26, M⁺ - H₂O), 394 (13, M⁺ - 2H₂O), 379 (24, M⁺ - CH₃ - 2H₂O), 351 (20), 315 (27), 293 (34), 259 (43), 173 (94), 149 (72), 135 (100); MS (ESI) *m*/*z* 453 (95, [M+Na]⁺), 883 (50, [2M+Na]⁺), 1314 (10, [3M+H+Na]⁺); exact mass calculated for C₂₈H₄₆O₃Na [M+Na]⁺ 453.3345 found 453.3358.

### Comparative (20R,22R)-2-Methylene-19-nor-22-methyl-1α,25-dihydroxyvitamin D₃ (IID)

Phenyllithium (1.8 M in di-n-butyl ether, 60 µL, 9.08 mg, 108 µmol) was added to a stirred solution of the phosphine oxide **20** (54 mg, 93 µmol) in anhydrous THF (500 µL) at -30 °C. After 30 min the mixture was cooled to -78 °C and a precooled solution of the ketone **30B** (19 mg, 47 µmol) in anhydrous THF (300 + 200 µL) was added. The reaction mixture was stirred under argon at -78 °C for 4 hours and then at +4 °C for 19 h. Ethyl acetate was added and the organic phase was washed with brine, dried (Na₂SO₄) and concentrated. The residue was applied to a Waters silica Sep-Pak cartridge (5 g). The cartridge was washed with hexane and ethyl acetate/hexane (1:99) to give the protected vitamin **31B** (32.64 mg, 91% yield).
UV (in hexane) λmax 262.5, 253.0, 245.0 nm; ¹H NMR (500 MHz, CDCl₃) δ 6.22 (1H, d, J = 11.2 Hz, 6-H), 5.84 (1H, d, J = 11.2 Hz, 7-H), 4.97 (1H, s, =CH₂), 4.92 (1H, s, =CH₂), 4.43 (2H, m, 1β-H and 3α-H), 2.82 (1H, dm, J = 12.4 Hz), 2.53 (1H, dd, J = 13.3, 5.9 Hz, 10α-H), 2.47 (1H, dd, J= 12.8, 4.5 Hz, 4α-H), 2.32 (1H, dd, J = 13.3, 2.9 Hz, 10β-H), 2.18 (1H, dd, J = 12.8, 8.4 Hz, 4β-H), 1.204 and 1.182 (each 3H, each s, 26-H₃, 27-H₃), 0.955 (9H, t, J = 7.9 Hz), 0.898 (9H, s, t-BuSi), 0.863 (9H, s, t-BuSi), 0.858 (3H, d, J = 5.4 Hz, 21-H₃), 0.808 (3H, d, J = 6.8 Hz), 0.569 (6H, q, J = 7.9 Hz), 0.542 (3H, s, 18-H₃), 0.081 (3H, s, SiMe), 0.065 (3H, s, SiMe), 0.050 (3H, s, SiMe), 0.024 (3H, s, SiMe); ¹³C NMR (125 MHz, CDCl₃) δ 152.99 (0, C-2), 141.36 (0, C-8), 132.71 (0, C-5), 122.43 (1, C-6), 116.05 (1, C-7), 106.25 (2, =CH₂), 73.58 (0, C-25), 72.56 and 71.60 (each 1, C-1, C-3), 56.31 (1), 54.28 (1), 47.62 (2), 45.62 (0, C-13), 44.01 (2), 41.94 (1), 40.73 (2), 38.53 (2), 35.41 (1), 30.40 and 29.50 (each 3, C-26, C-27), 28.81 (2), 27.46 (2), 25.84 (3), 25.78 (3), 23.70 (2), 23.49 (2), 22.13 (2), 19.01 (3), 18.26 (0), 18.16 (0), 13.11 (3), 11.97 (3), 7.16 (3), 6.86 (2), -4.86 (3), -4.91 (3), -5.11 (3); MS (ESI) *m*/*z* 795 (50, [M+Na⁺]); exact mass (ESI) calculated for C₄₆H₈₈O₃Si₃Na [M+Na]⁺ 795.5939, found 795.5916.

The protected vitamin **31B** (32.64 mg, 42 µmol) was dissolved in THF (4 mL) and acetonitrile (3 mL). A solution of aqueous 48% HF in acetonitrile (1:9 ratio, 4 mL) was added at 0 °C and the resulting mixture was stirred at room temperature for 2 h. Saturated aqueous NaHCO₃ solution was added and the reaction mixture was extracted with dichloromethane. The combined organic phases were dried (Na₂SO₄) and concentrated under reduced pressure. The residue was diluted with 2 mL of hexane/ethyl acetate (7:3) and applied to a Waters silica Sep-Pak cartridge (5 g). An elution with hexane/ethyl acetate (7:3, then 1:1) gave the crude product **IID.** The vitamin **IID** was further purified by straight phase HPLC [9.4 x 250 mm Zorbax Silica column, 5 mL/min, hexane/2-propanol (85:15) solvent system, Rₜ 6.5 min.] and reverse phase HPLC [9.4 x 250 mm Zorbax RX-C18 column, 3 mL/min, methanol/water (85:15) solvent system, Rₜ 13.2 min.] to give the pure comparative compound **IID** (15.28 mg, 78% yield). Pure crystals of the analog **IID** were obtained after crystallization from hexane/2-propanol and they were characterized by an X-ray analysis.
m.p. 159 °C (hexane/2-propanol); UV (in EtOH) λmax 261.5, 252.5, 244.5 nm; ¹H NMR (500 MHz, CDCl₃) δ 6.35 (1H, d, J = 11.3 Hz, 6-H), 5.89 (1H, d, J = 11.3 Hz, 7-H), 5.11 (1H, s, =CH₂), 5.08 (1H, s, =CH₂), 4.46 (2H, m, 1β-H and 3α-H), 2.85 (1H, dd, J = 13.2, 4.5 Hz, 10β-H), 2.83 (1H, dm, J = 13.6 Hz, 9β-H), 2.57 (1H, dd, J = 13.4, 3.8 Hz, 4α-H), 2.33 (1H, dd, J = 13.4, 6.1 Hz, 4β-H), 2.29 (1H, dd, J = 13.2, 8.4 Hz, 10α-H), 1.227 and 1.219 (each 3H, each s, 26-H₃, 27-H₃), 0.882 (3H, d, J = 6.8 Hz, 21-H₃), 0.818 (3H, d, J = 6.8 Hz, 28-H₃), 0.549 (3H, s, 18-H₃); ¹³C NMR (125 MHz, CDCl₃) δ 151.97 (0, C-2), 143.39 (0, C-8), 130.44 (0, C-5), 124.19 (1, C-6), 115.25 (1, C-7), 107.69 (2, =CH₂), 71.23 (0, C-25), 71.78 and 70.59 (each 1, C-1, C-3), 56.25 (1), 54.15 (1), 45.74 (2), 45.74 (0), 42.76 (2), 41.79 (1), 40.50 (2), 38.12 (2), 35.15 (1), 29.53 (3), 29.01 (3), 29.01 (2), 27.35 (2), 23.66 (2), 23.52 (2), 22.19 (2), 18.93 (3), 13.13 (3), 12.02 (3); MS (EI) m/z 430 (100, M⁺), 412 (24, M⁺ - H₂O), 394 (10, M⁺ - 2H₂O), 379 (10, M⁺ - CH₃ - 2H₂O), 343 (9), 315 (41), 297 (26), 262 (53), 183 (21), 161 (30), 135 (50); exact mass (ESI) calculated for C₂₈H₄₆O₃ [M+Na]⁺ 453.3345 found 453.3344.

### EXAMPLE 2: BIOLOGICAL ACTIVITY

### Vitamin D Receptor Binding

### Test Material

### Protein Source

Full-length recombinant rat receptor was expressed in *E. coli* BL21(DE3) Codon Plus RIL cells and purified to homogeneity using two different column chromatography systems. The first system was a nickel affinity resin that utilizes the C-terminal histidine tag on this protein. The protein that was eluted from this resin was further purified using ion exchange chromatography (S-Sepharose Fast Flow). Aliquots of the purified protein were quick frozen in liquid nitrogen and stored at -80°C until use. For use in binding assays, the protein was diluted in TEDK₅₀ (50 mM Tris, 1.5 mM EDTA, pH 7.4, 5 mM DTT, 150 mM KCl) with 0.1% Chaps detergent. The receptor protein and ligand concentration was optimized such that no more than 20% of the added radiolabeled ligand was bound to the receptor.

### Study Drugs

Unlabeled ligands were dissolved in ethanol and the concentrations determined using UV spectrophotometry (1,25(OH)₂D₃: molar extinction coefficient = 18,200 and λₘₐₓ = 265 nm; Analogs: molar extinction coefficient = 42,000 and λₘₐₓ = 252 nm). Radiolabeled ligand (³H-1,25(OH)₂D₃, ∼159 Ci/mmole) was added in ethanol at a final concentration of 1 nM.

### Assay Conditions

Radiolabeled and unlabeled ligands were added to 100 mcl of the diluted protein at a final ethanol concentration of ≤ 10%, mixed and incubated overnight on ice to reach binding equilibrium. The following day, 100 mcl of hydroxylapatite slurry (50%) was added to each tube and mixed at 10-minute intervals for 30 minutes. The hydroxylapaptite was collected by centrifugation and then washed three times with Tris-EDTA buffer (50 mM Tris, 1.5 mM EDTA, pH 7.4) containing 0.5% Titron X-100. After the final wash, the pellets were transferred to scintillation vials containing 4 ml of Biosafe II scintillation cocktail, mixed and placed in a scintillation counter. Total binding was determined from the tubes containing only radiolabeled ligand.

### HL-60 Differentiation

### Test Material

### Study Drugs

The study drugs were dissolved in ethanol and the concentrations determined using UV spectrophotometry. Serial dilutions were prepared so that a range of drug concentrations could be tested without changing the final concentration of ethanol (≤ 0.2%) present in the cell cultures.

### Cells

Human promyelocytic leukemia (HL60) cells were grown in RPMI-1640 medium containing 10% fetal bovine serum. The cells were incubated at 37°C in the presence of 5% CO₂.

### Assay Conditions

HL60 cells were plated at 1.2 x 10⁵ cells/ml. Eighteen hours after plating, cells in duplicate were treated with drug. Four days later, the cells were harvested and a nitro blue tetrazolium reduction assay was performed (Collins et al., 1979; J. Exp. Med. 149:969-974). The percentage of differentiated cells was determined by counting a total of 200 cells and recording the number that contained intracellular black-blue formazan deposits. Verification of differentiation to monocytic cells was determined by measuring phagocytic activity (data not shown).

### In Vitro Transcription Assay

Transcription activity was measured in ROS 17/2.8 (bone) cells that were stably transfected with a 24-hydroxylase (24OHase) gene promoter upstream of a luciferase reporter gene (Arbour *et al.,* 1998). Cells were given a range of doses. Sixteen hours after dosing the cells were harvested and luciferase activities were measured using a luminometer. RLU = relative luciferase units.

### Intestinal Calcium Transport and Bone Calcium Mobilization

Male, weanling Sprague-Dawley rats were placed on Diet 11 (0.47% Ca) diet + AEK oil for one week followed by Diet 11 (0.02% Ca) + AEK oil for 3 weeks. The rats were then switched to a diet containing 0.47% Ca for one week followed by two weeks on a diet containing 0.02% Ca. Dose administration began during the last week on 0.02% calcium diet. Four consecutive intraperitoneal doses were given approximately 24 hours apart. Twenty-four hours after the last dose, blood was collected from the severed neck and the concentration of serum calcium determined as a measure of bone calcium mobilization. The first 10 cm of the intestine was also collected for intestinal calcium transport analysis using the everted gut sac method.

### Biological Activity Results

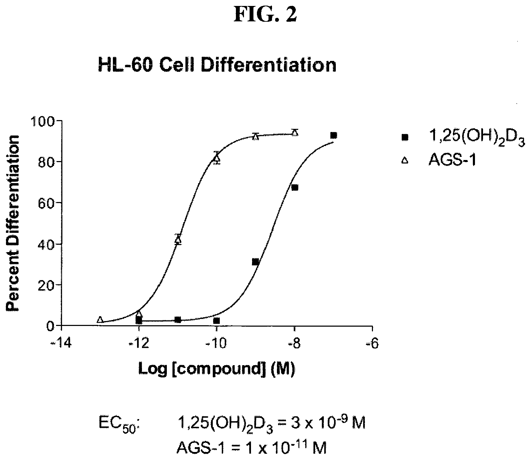

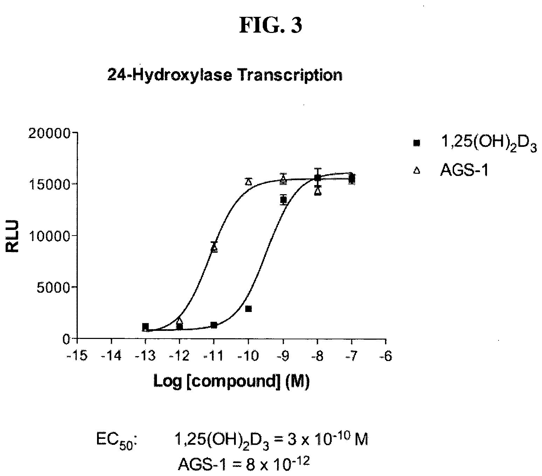

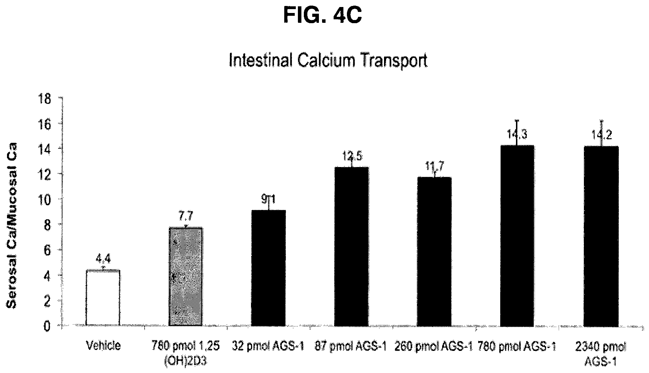

(20S, 22R)-2-Methylene-19-nor-22-methyl-1α,25-dihydroxyvitamin D₃ (AGS-1) is approximately equally effective as 1,25-(OH)₂D₃ in binding to the recombinant vitamin D receptor as shown in Fig. 1. However, it is substantially more potent (∼300 times) than 1,25-(OH)₂D₃ in causing the differentiation of HL-60 cells in culture (Fig. 2). Likewise, it is nearly 40 times more active than 1,25-(OH)₂D₃ in increasing transcription of the 24-hydroxylase gene (Fig. *3**). In vivo* testing demonstrated that this compound is more potent than 1,25-(OH)₂D₃ in promoting both bone calcium mobilization (Figs. 4A and 4B) and intestinal calcium transport (Fig. 4C). Because AGS-1 is dramatically more potent than the native hormone in causing cellular differentiation and has a unique ability to stimulate bone calcium mobilization to a greater level than the native hormone, it may serve as a useful therapy for various bone diseases.

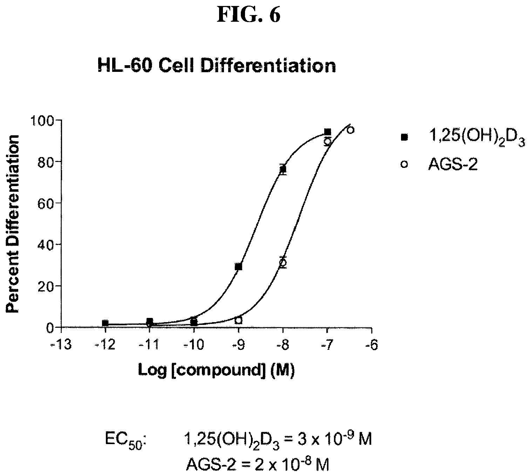

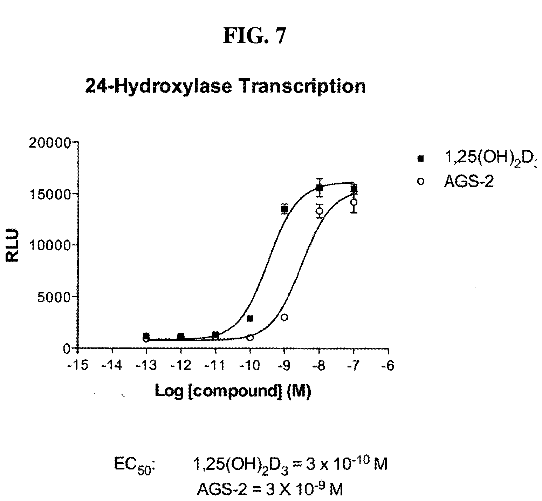

On the other hand, (20S, 22S)-2-methylene-19-nor-22-methyl-1α,25-dihydroxyvitamin D₃ (AGS-2) showed lower affinity relative to 1,25-(OH)₂D₃ in binding to the recombinant vitamin D receptor as shown in Fig. 5. Nonetheless, it possesses significant cell differentiation and transcription activity. It is only about 10 times less active than 1,25-(OH)₂D₃ in causing the differentiation of HL-60 cells in culture (Fig. 6). Likewise, it is about 10 times less active than 1,25-(OH)₂D₃ in increasing transcription of the 24-hydroxylase gene (Fig. 7). *In vivo* testing demonstrated that AGS-2 has a much reduced ability to mobilize calcium from bone compared to 1,25-(OH)₂D₃ (Fig. 8A). However, its intestinal calcium transport activity is similar or greater than 1,25-(OH)₂D₃ (Fig. 8B). The intestinal specific nature of AGS-2 coupled with its cellular differentiation activity make it a candidate for therapy in intestinal based diseases, such as Crohn's disease or celiac disease. Further, these compounds should find utility in the treatment of secondary hyperparathyroidism of patients suffering from chronic kidney failure because it is undesirable to elevate serum calcium above normal in these patients for fear of calcification of heart, aorta and other vital organs while suppressing parathyroid gland proliferation and transcription of the preproparathyroid gene. Likewise, these compounds should also be useful in the treatment of malignancy such as breast, colorectal and prostate cancers, or in the treatment of autoimmune diseases such as multiple sclerosis, lupus, rheumatoid arthritis, type 1 diabetes, and inflammatory bowel disease. They should also be useful in preventing transplant rejection.

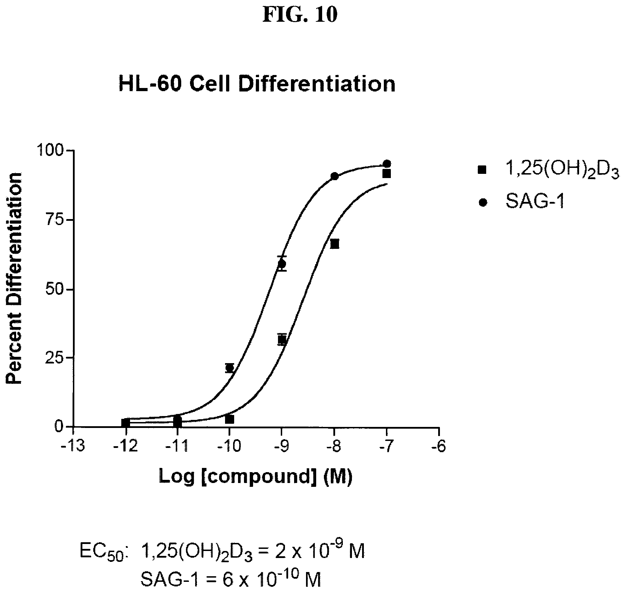

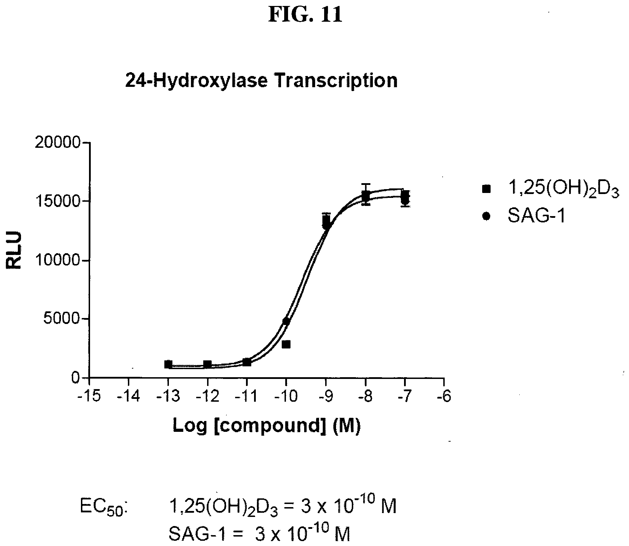

Comparative compound (20R, 22S)-2-Methylene-19-nor-22-methyl-1α,25-dihydroxyvitamin D₃ (SAG-1) is similar or slightly less effective than 1,25-(OH)₂D₃ in binding to the recombinant vitamin D receptor as shown in comparative Fig. 9. However, it is more potent (>3 times) than 1,25-(OH)₂D₃ in causing the differentiation of HL-60 cells in culture (comparative Fig. 10). It is similar to 1,25-(OH)₂D₃ in increasing transcription of the 24-hydroxylase gene (comparative Fig. 11), suggesting that there may be some cell-specific differences with comparative compound SAG-1. *In vivo* testing demonstrated that this compound is less potent than 1,25-(OH)₂D₃ in promoting bone calcium mobilization (comparative Figs. 12A and 12B) and is of similar potency to 1,25-(OH)₂D₃ in intestinal calcium transport (comparative Figs. 12C and 12D). Thus, comparative compound SAG-1 has a biological activity profile indicating that it possesses cell specific activity and *in vivo* shows that it would likely have a larger therapeutic index compared to the native hormone.

Comparative compound (20R, 22R)-2-Methylene-19-nor-22-methyl-1α,25-dihydroxyvitamin D₃ (SAG-2) is less effective than 1,25-(OH)₂D₃ in binding to the recombinant vitamin D receptor as shown in comparative Fig. 13. It is also less potent (∼3 times) than 1,25-(OH)₂D₃ in causing the differentiation of HL-60 cells in culture (comparative Fig. 14). It is approximately 20 times less potent than 1,25-(OH)₂D₃ in causing transcription of the 24-hydroxylase gene (comparative Fig. 15). *In vivo* testing demonstrated that this compound is markedly lower than 1,25-(OH)₂D₃ both with respect to promoting bone calcium mobilization (comparative Figs. 16A and 16B) and in intestinal calcium transport (comparative Figs. 16C and 16D). Thus, comparative compound SAG-2 has a biological activity profile indicating that it might possess overall reduced potency, but a larger therapeutic index compared to the native hormone.

Comparative Example: Table 1 shows biological data for the compounds from the present disclosure (AGS-1, AGS-2) and for comparative compounds (SAG-1, and SAG-2) in comparison to 2-methylene-19-nor-1α,25-dihydroxyvitamin D₃ and its 20R isomer. AGS-1, AGS-2, SAG-1 and SAG-2 differ from 2-methylene-19-nor-1α,25-dihydroxyvitamin D₃ and its 20R isomer in that they have a methyl group attached to the position 21 carbon. The present AGS compounds and the comparative SAG compounds display surprising and unexpected bioactivity in comparison to the 2MD compounds. The 2MD compounds show extremely potent net bone calcium mobilization activity (ranging from 4.5 mg/dL in the 20R isomer to 9.3 mg/dL in the 20S isomer). In stark contrast, AGS-2, comparative compound SAG-1, and comparative compound SAG-2 all show no net calcemic activity on bone. While AGS-1 does show activity with regard to net bone calcium mobilization, this compound *also* shows significant activity on net intestinal calcium transport (serosal to mucosal ratio of 4.3) unlike the 2MD compounds, which demonstrate intestinal calcemic activity lower than that of vehicle (serosal to mucosal ratios of -0.6 for the 20R isomer and -0.9 for the 20S isomer). Likewise, AGS-2 displays significant net intestinal calcium transport, in contrast to the 2MD compounds. Thus, AGS-2 displays a calcemic activity profile opposite to that of the 2MD compounds. AGS-1 is further differentiated from the 2MD compounds in the HL-60 assay results. In particular, while the 2MD compounds are approximately 1 to 27 times more active than the native hormone in HL-60 differentiation, AGS-1 is -300 times more active than the native hormone. Thus, AGS-1 is at least 10 time more active than the 20S isomer of 2MD (i.e., 300/27 ≈ 11) and more than 300 times more active than the 20R isomer of 2MD (i.e., 300/0.95 ≈ 320).

There is also provided the compounds of the present technology for use in preventing or treating obesity, inhibiting adipocyte differentiations, inhibiting SCD-1 gene transcription, and/or reducing body fat in animal subjects. Therefore, in some embodiments, there is provided the compounds of the present technology for use in preventing or treating obesity, inhibiting adipocyte differentiations, inhibiting SCD-1 gene transcription, and or reducing body fat in animal subject includes administering to the animal subject, an effective amount of the compound or a pharmaceutical composition that includes the compound. Administration of the compound or the pharmaceutical composition to the subject inhibits adipocyte differentiation, inhibits gene transcription, and/or reduces body fat in the animal subject.

**Table 1**

| **Working Example¹** | **Where** | **Side chain** | **Competitive VDR Binding²** | **HL-60 Differentiation³** | **24OHase Transcription³** | **Net Bone Ca²⁺ Mobilization⁵** | **Net Intestinal Ca²⁺ Transport⁶** |
|---|---|---|---|---|---|---|---|
| | | | | **(Relative Activity⁴)** | **(Relative Activity⁴)** | | |
| AGS-1 | Present | 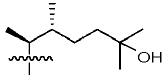 | 0.07 | 0.01 (300) | 0.008 (38) | 5.3 | 9.9 |
| AGS-2 | Present | 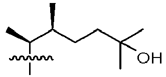 | 8 | 20 (0.15) | 3 (0.1) | 0 | 4.3 |
| SAG-1 (Comparative) | Present | 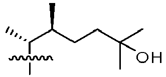 | 0.09 | 0.6 (3.3) | 0.3 (10) | 0.3⁷ | 1.3⁷ |
| SAG-2 (Comparative) | Present | 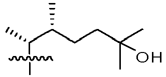 | 0.2 | 9 (0.3) | 6 (0.05) | -0.1⁷ | 0.4⁷ |
| 2MD⁸ (20R) | US 5,843,928 | 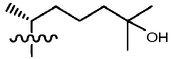 | 0.12 | 4.2 (0.95) | - | 4.5⁷ | -0.6⁷ |
| 2MD⁸ (20S) | US 5,843,928 | 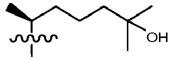 | 0.10 | 0.15 (27) | - | 9.3⁷ | -0.9⁷ |

| | | | | | | | |
|---|---|---|---|---|---|---|---|
| ¹ All compounds are 2-methylene 19-nor compounds. ² Kᵢ, nM. ³ EC₅₀, nM. ⁴ Activity relative to the native hormone, 1,25(OH)₂D₃, as measured in the same assay. Relative activity = (value observed for native hormone) / (value observed for working example). Ratios greater than one indicate the working example is more active than the native hormone. ⁵ In mg/dL at 780 pM dosage, except where indicated. ⁶ Serosal Ca²⁺ to mucosal Ca²⁺ ratio, S/M, at 780 pM dosage, except where indicated. ⁷ At 260 pM dosage. ⁸ Data from US 5,843,928 and *J*. *Med. Chem.* 1998, 41, 4662. | | | | | | | |

## Claims

1. A compound having the formula IA or IB 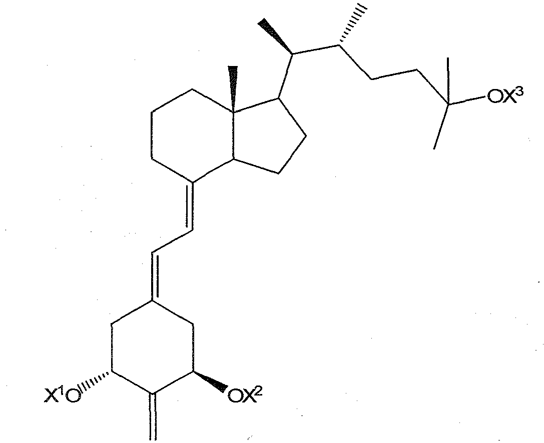 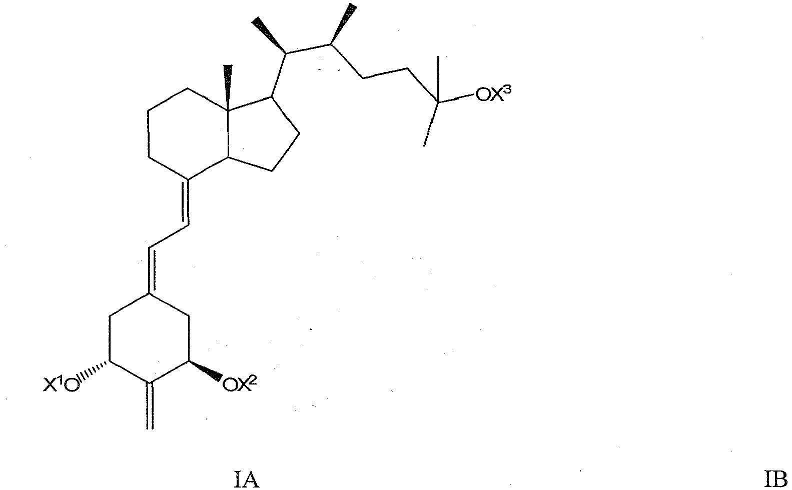 wherein
X¹ and X² are independently selected from H and hydroxy protecting groups; and
X³ is H.

2. The compound of claim 1, wherein X¹ and X² are hydroxy protecting groups or wherein X¹ and X² are both t-butyldimethylsilyl groups.

3. The compound of claim 1, having the formula IIA or IIB

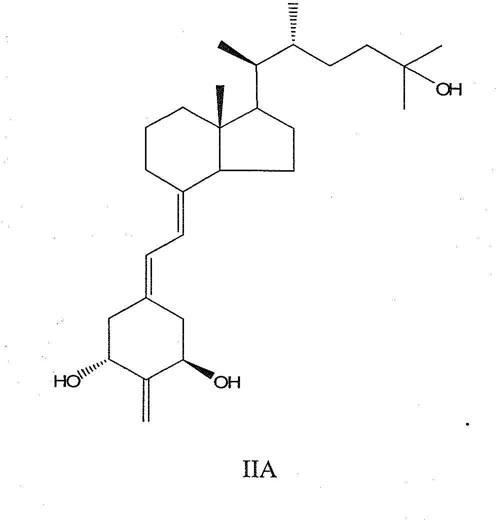

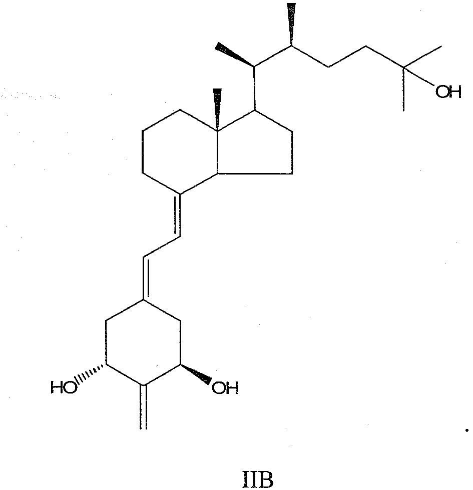

4. The compound of claim 3, having the formula IIE or IIF

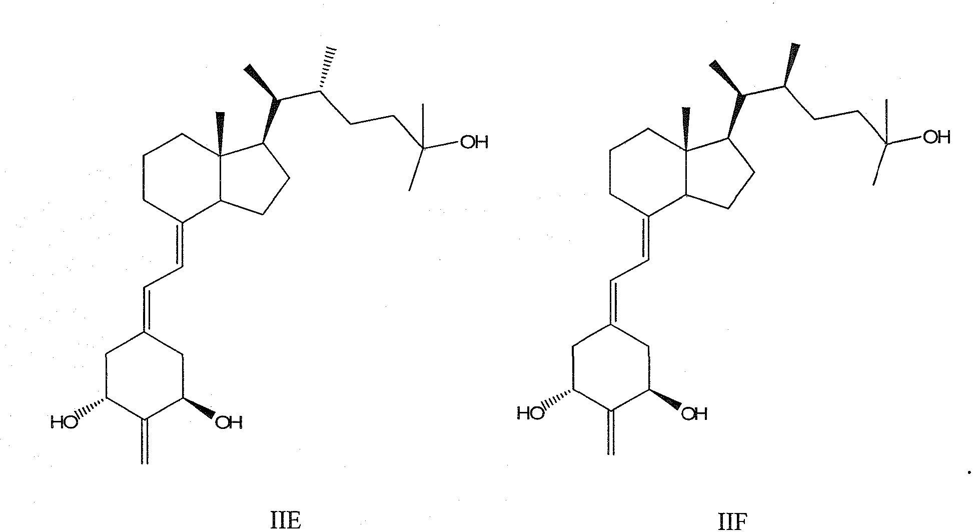

5. A pharmaceutical composition, comprising an effective amount of the compound of claim 3 or claim 4 and a pharmaceutically acceptable carrier.

6. The pharmaceutical composition of claim 5 wherein the effective amount comprises from 0.01 µg to 1 mg of the compound per gram of the composition, generally from 0.1 µg to 500 µg of the compound per gram of the composition.

7. The compound of claim 3 or claim 4 for use in the treatment of a subject suffering from a biological condition, wherein the biological condition is selected from psoriasis; leukemia; colon cancer; breast cancer; prostate cancer; multiple sclerosis; lupus; diabetes mellitus; host versus graft reaction; rejection of organ transplants; an inflammatory disease selected from rheumatoid arthritis, asthma, or inflammatory bowel diseases; a skin condition selected from lack of adequate dermal hydration or insufficient sebum secretion; renal osteodystrophy; or osteoporosis.

8. The compound for use of claim 7, wherein the biological condition is psoriasis, lack of adequate dermal hydration, or insufficient sebum secretion.

9. The compound for use of claim 7, wherein the biological condition is selected from leukemia, colon cancer, breast cancer, or prostate cancer.

10. The compound for use of claim 7, wherein the biological condition is selected from multiple sclerosis, lupus, diabetes mellitus host versus graft reaction, or rejection of organ transplants.

11. The compound for use of claim 7, wherein the biological condition is selected from rheumatoid arthritis, asthma, or inflammatory bowel diseases selected from celiac disease, ulcerative colitis and Crohn's disease.

12. The compound for use of claim 7, wherein the compound is administered orally, parenterally, transdermally, or topically to the subject.

13. The compound for use of claim 7, wherein the compound is administered in a dosage of from 0.01 µg per day to 1 mg per day.

14. A method comprising administering an effective amount of the compound of claim 3 or claim 4 to the subject suffering from wrinkles or lack of adequate skin firmness.

15. The method of claim 14, wherein the compound is administered topically to the subject.

## Patentansprüche

1. Verbindung der Formel IA oder IB, 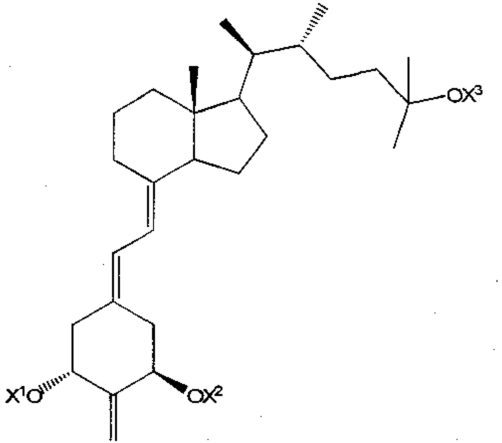 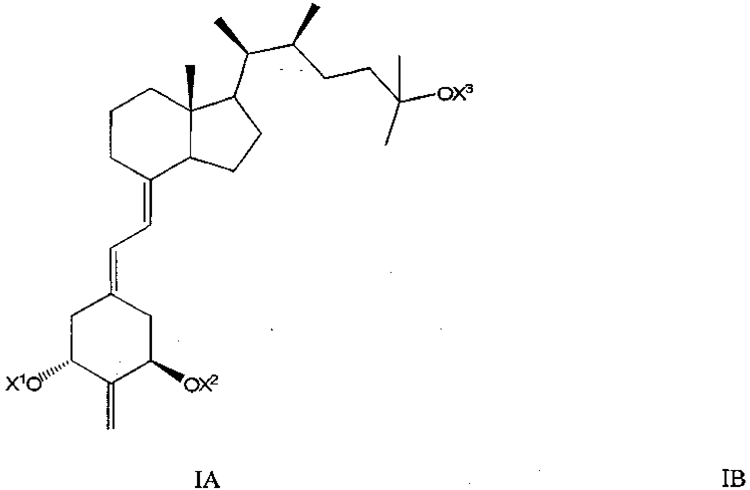 wobei
X¹ und X² unabhängig aus H und Hydroxy-Schutzgruppen ausgewählt werden; und
X³ H ist.

2. Verbindung nach Anspruch 1, wobei X¹ und X² Hydroxy-Schutzgruppen sind oder wobei X¹ und X² beide t-Butyldimethylsilyl-Gruppen sind.

3. Verbindung nach Anspruch 1 der Formel IIA oder IIB

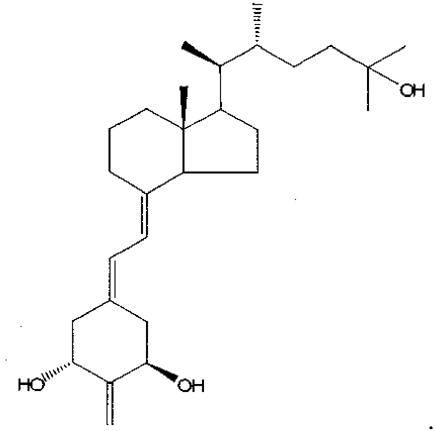

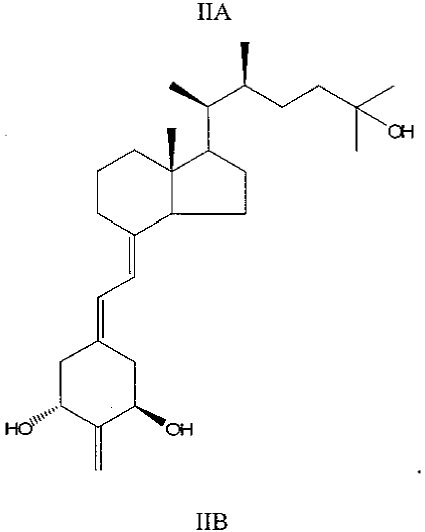

4. Verbindung nach Anspruch 3 der Formel IIE oder IIF

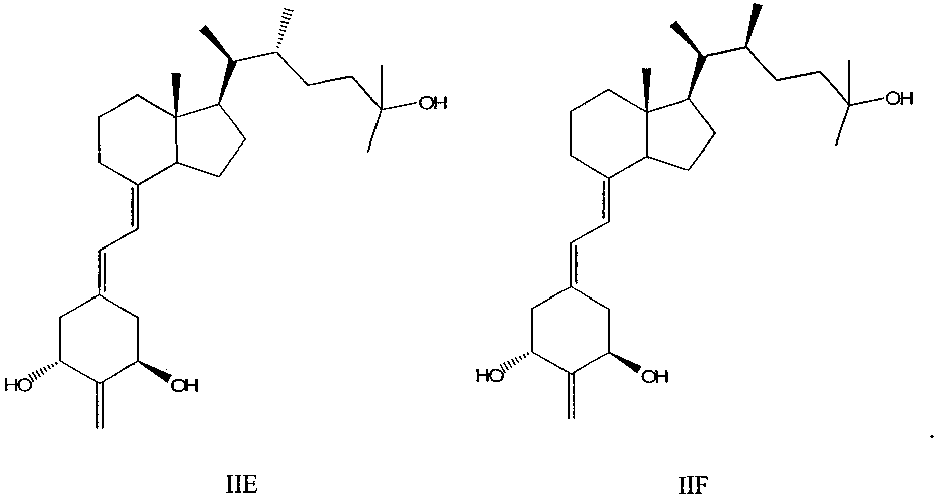

5. Pharmazeutische Zusammensetzung, umfassend eine wirksame Menge der Verbindung nach Anspruch 3 oder Anspruch 4 und einen pharmazeutisch unbedenklichen Trägerstoff.

6. Pharmazeutische Zusammensetzungen nach Anspruch 5, wobei die wirksame Menge von 0,01 µg bis 1 mg der Verbindung je Gramm der Zusammensetzung umfasst, im Allgemeinen von 0,1 µg bis 500 µg der Verbindung je Gramm der Zusammensetzung.

7. Verbindung nach Anspruch 3 oder Anspruch 4 zur Verwendung in der Behandlung einer Person, die an einem biologischen Zustand leidet, wobei der biologische Zustand aus Schuppenflechte; Leukämie; Darmkrebs; Brustkrebs; Prostatakrebs; Multipler Sklerose; Lupus; Diabetes mellitus; einer Wirt-anti-Transplantat-Reaktion; einer Abstoßung von Organtransplantaten; einer Entzündungserkrankung, die aus rheumatoider Arthritis, Asthma, oder entzündlichen Darmerkrankungen ausgewählt ist; einem Hautzustand, der aus einem Fehlen von angemessener Wasseraufnahme der Haut oder ungenügender Hauttalgabsonderung ausgewählt ist; renaler Osteodystrophie oder Osteoporose ausgewählt ist.

8. Verbindung zur Verwendung nach Anspruch 7, wobei der biologische Zustand Schuppenflechte, Fehlen von angemessener Wasseraufnahme der Haut oder ungenügende Hauttalgabsonderung ist.

9. Verbindung zur Verwendung nach Anspruch 7, wobei der biologische Zustand aus Leukämie, Darmkrebs, Brustkrebs oder Prostatakrebs ausgewählt ist.

10. Verbindung zur Verwendung nach Anspruch 7, wobei der biologische Zustand aus multipler Sklerose, Lupus, Diabetes mellitus, einer Wirt-anti-Transplantat-Reaktion oder einer Abstoßung von Organtransplantaten ausgewählt ist.

11. Verbindung zur Verwendung nach Anspruch 7, wobei der biologische Zustand aus rheumatoider Arthritis, Asthma oder entzündlichen Darmerkrankungen ausgewählt ist, die aus Zöliakie, Colitis ulcerosa und Morbus Crohn ausgewählt sind.

12. Verbindung zur Verwendung nach Anspruch 7, wobei die Verbindung der Person oral, parenteral, transdermal oder lokal verabreicht wird.

13. Verbindung zur Verwendung nach Anspruch 7, wobei die Verbindung in einer Dosierung von 0,01 µg pro Tag bis 1 mg pro Tag verabreicht wird.

14. Verfahren, umfassend Verabreichen einer wirksamen Menge der Verbindung nach Anspruch 3 oder Anspruch 4 an die Person, die an Falten oder Fehlen angemessener Hautfestigkeit leidet.

15. Verfahren nach Anspruch 14, wobei die Verbindung der Person lokal verabreicht wird.

## Revendications

1. Composé représenté par la formule IA ou IB 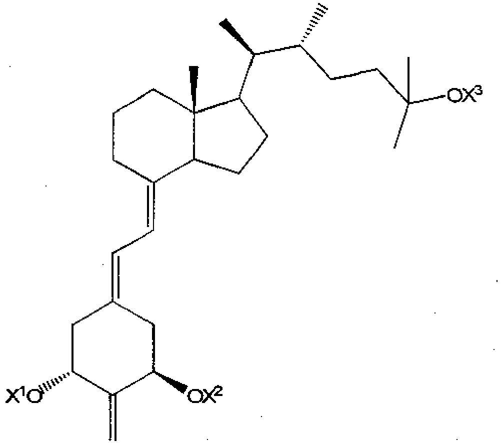 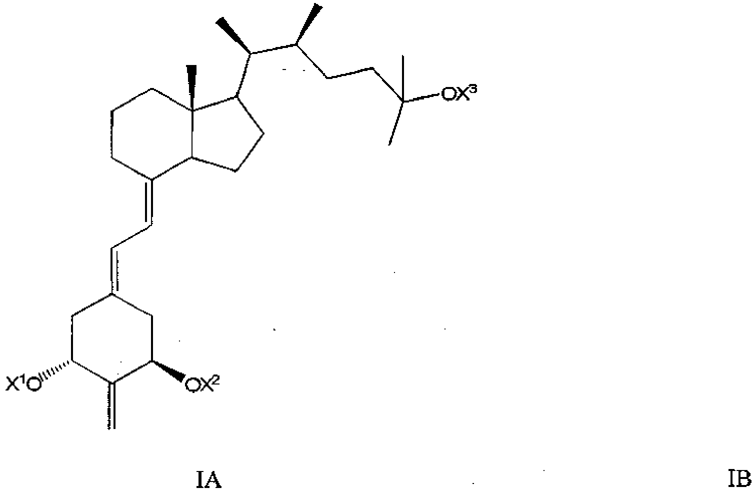 dans lesquelles
X¹ et X² sont indépendamment choisis parmi l'atome H et des groupes protecteurs hydroxy ; et
X³ représente un atome H.

2. Composé selon la revendication 1, X¹ et X² représentant des groupes protecteurs hydroxy ou X¹ et X² représentant tous les deux des groupes t-butyldiméthylsilyle.

3. Composé selon la revendication 1, représenté par la formule IIA ou IIB

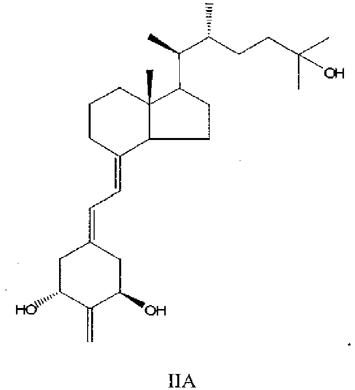

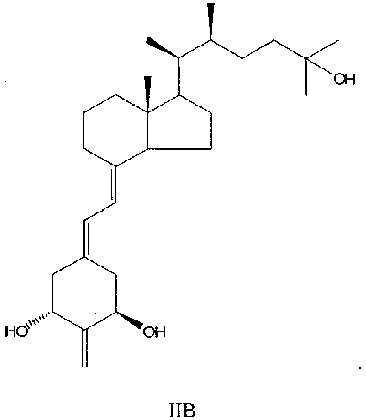

4. Composé selon la revendication 3, représenté par la formule IIE ou IIF

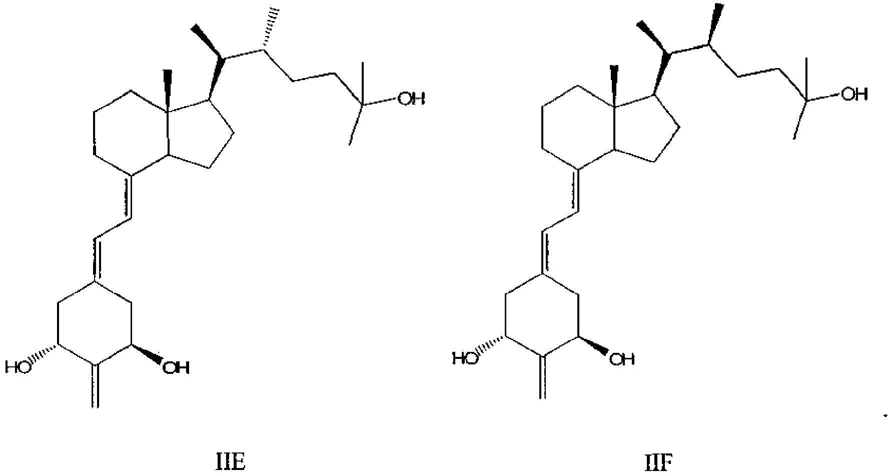

5. Composition pharmaceutique, comprenant une quantité efficace du composé selon la revendication 3 ou 4 et un véhicule pharmaceutiquement acceptable.

6. Composition pharmaceutique selon la revendication 5, ladite quantité efficace comprenant de 0,01 µg à 1 mg du composé par gramme de la composition, généralement de 0,1 µg à 500 µg du composé par gramme de la composition.

7. Composé selon la revendication 3 ou 4 destiné à être utilisé dans le traitement d'un sujet souffrant d'une affection biologique, ladite affection biologique étant choisie parmi le psoriasis ; la leucémie ; le cancer du côlon ; le cancer du sein ; le cancer de la prostate ; la sclérose en plaques ; le lupus ; le diabète sucré ; la réaction de l'hôte contre le greffon ; le rejet de greffes d'organes ; une maladie inflammatoire choisie parmi par la polyarthrite rhumatoïde, l'asthme ou des maladies intestinales inflammatoires ; un état dermatologique choisi parmi le manque d'hydratation dermique adéquate ou une sécrétion de sébum insuffisante ; l'ostéodystrophie rénale ; ou l'ostéoporose.

8. Composé destiné à être utilisé selon la revendication 7, ladite affection biologique étant le psoriasis, le manque d'hydratation dermique adéquate ou une sécrétion de sébum insuffisante.

9. Composé destiné à être utilisé selon la revendication 7, ladite affection biologique étant choisie parmi la leucémie, le cancer du côlon, le cancer du sein ou le cancer de la prostate.

10. Composé destiné à être utilisé selon la revendication 7, ladite affection biologique étant choisie parmi la sclérose en plaques, le lupus, le diabète sucré, la réaction de l'hôte contre le greffon ou le rejet de greffes d'organes.

11. Composé destiné à être utilisé selon la revendication 7, ladite affection biologique étant choisie parmi la polyarthrite rhumatoïde, l'asthme ou des maladies intestinales inflammatoires choisies parmi la maladie coeliaque, la colite ulcéreuse et la maladie de Crohn.

12. Composé destiné à être utilisé selon la revendication 7, ledit composé étant administré par voie orale, parentérale, transdermique ou topique au sujet.

13. Composé destiné à être utilisé selon la revendication 7, ledit composé étant administré selon une posologie allant de 0,01 µg par jour à 1 mg par jour.

14. Méthode comprenant l'administration d'une quantité efficace du composé selon la revendication 3 ou 4 au sujet souffrant de rides ou d'un manque de fermeté adéquate de la peau.

15. Méthode selon la revendication 14, ledit composé étant administré par voie topique au sujet.
